# EUROPEAN PATENT APPLICATION

(11) **EP 3 998 355 A1**
(43) Date of publication of application: **18.05.2022**
(21) Application number: 20837705.1
(22) Date of filing: 30.06.2020
(51) Int. Cl.: C12Q 1/6895, C12Q 1/6886

(54) **METHOD FOR DIAGNOSING BRAIN TUMOR THROUGH BACTERIAL METAGENOMIC ANALYSIS**

(30) Priority: 08.07.2019 KR 20190082317; 14.05.2020 KR 20200057634
(71) Applicant: MD Healthcare Inc., Seoul 03923 (KR); Seoul National University R & DB Foundation, Seoul 08826 (KR)
(72) Inventor: KIM, Yoon-Keun, Paju-Si, Gyeonggi-do 10908 (KR); PAEK, Sun Ha, Seoul 03080 (KR); MOON, Hyo Eun, Seoul 03080 (KR); PARK, Hyung Woo, Seoul 03080 (KR)
(74) Representative: Dehns
(86) International application number: PCT/KR2020/008508
(87) International publication number: WO 2021/006523

(57) **Abstract**

The present invention relates to a method of diagnosing a brain tumor through bacterial metagenomic analysis, and more particularly, to a method of diagnosing a brain tumor by analyzing the increase or decrease in content of specific bacteria-derived extracellular vesicles by performing bacterial metagenomic analysis using a normal individual and a subject-derived sample. By diagnosing the risk of the onset of a brain tumor through the metagenomic analysis of bacteria-derived extracellular vesicles using a human-derived sample according to the present invention, it is possible to diagnose and predict a risk group for a brain tumor early, and also to delay or prevent the onset of the disease through appropriate management. In addition, there are advantages that, even after the onset of a brain tumor, early diagnosis can be performed to reduce the incidence of the brain tumor, a therapeutic effect cannot be only increased, but also the disease can be improved and recurrence thereof can be prevented by avoiding exposure to causative factors through metagenomic analysis in patients diagnosed with a brain tumor.

## Description

### [Technical Field]

The present invention relates to a method of diagnosing a brain tumor through bacterial metagenomic analysis, and more particularly, to a method of diagnosing a brain tumor by analyzing the increase or decrease in content of specific bacteria-derived extracellular vesicles by performing bacterial metagenomic analysis using a normal individual and a subject-derived sample.

This application claims priority to and the benefit of Korean Patent Application Nos. 10-2019-0082317 and 10-2020-0057634 filed in the Korean Intellectual Property Office on July 8, 2019 and May 14, 2020, respectively, and all the contents disclosed in the specification and drawings of the applications are incorporated in this application.

### [Background Art]

A brain tumor is a tumor generated in the brain and the central nervous system, and may be used interchangeably with "brain cancer." Generally, a brain tumor is the fourth or fifth most common tumor of tumors occurring throughout the body, and especially, the second most common tumor among all malignant tumors occurring in children. The incidence of brain tumors is about 10 per 100,000 people, and a glioma, the most common primary brain tumor, accounts for about 35% of all primary brain tumors and is generally malignant. A mutagenic substance known so far as one of the causes of brain tumors is radiation, a chemical or a virus, and even when an immune function is lowered like AIDS, it is known that the incidence of brain tumors increases. Recently, due to environmental pollution, interest in the relationship between a chemical substance and cancer is increasing, but a clear causal relationship with the onset of a brain tumor has not been established.

Mechanisms by which a brain tumor causes clinical symptoms include symptoms caused by increased intracranial pressure, symptoms caused by a brain tumor pressing on the brain around the tumor, symptoms caused by a brain tumor electrically stimulating surrounding brain nerves (epileptic seizure), and symptoms caused by hormonal changes. Without receiving active treatment, both malignant and benign brain tumors lead to fatal consequences.

As a method of determining the prognosis of existing brain tumor patients, generally, a general method of summarizing the progress of clinical treatment, and the progress of radiotherapy and chemotherapy, and determining a survival prognosis as well as possible recurrence by considering the severity and locus of a brain tumor and age was used, which has various differences depending on patient type, and different results for clinical treatment, and thus it may not be an accurate prognosis prediction method. Therefore, there is an emerging need for novel diagnosis for diagnosing a brain tumor and predicting the prognosis thereof, and development of a biomarker as a therapeutic target.

Meanwhile, a microbiota is a microbial community that includes bacteria, archaea, and eukaryotes present in a given habitat. The intestinal microbiota is known to play a vital role in human's physiological phenomena and significantly affect human health and diseases through interactions with human cells. Bacteria coexisting in human bodies secrete nanometer-sized vesicles to exchange information about genes, proteins, and the like with other cells. The mucous membranes form a physical barrier membrane that does not allow particles with the size of 200 nm or more to pass therethrough, and thus bacteria symbiotically living in the mucous membranes are unable to pass therethrough, but bacteria-derived extracellular vesicles have a size of approximately 100 nm or less and thus relatively freely pass through the mucous membranes and are absorbed into the human body.

Metagenomics, also called environmental genomics, may be analytics for metagenomic data obtained from samples collected from the environment. Recently, the bacterial composition of human microbiota has been listed using a method based on 16s ribosomal RNA (16s rRNA) base sequences, and 16s rDNAbase sequences, which are genes of 16s ribosomal RNA, are analyzed using a next generation sequencing (NGS) platform. However, in terms of brain tumor occurrence, a method of identifying causative factors of brain tumor through analysis of a metagenome present in the bacteria-derived vesicles from a human body-derived substance such as blood and predicting the brain tumor has not been reported yet.

### [Disclosure]

### [Technical Problem]

The inventors had extracted genes from bacteria-derived extracellular vesicles present in blood from a normal sample and a subject-derived sample, and conducted metagenomic analysis thereon in order to diagnose, in advance, causative factors of a brain tumor and the risk of the onset thereof, resulting in identification of bacteria-derived extracellular vesicles which can act as a causative factor of the brain tumor, and the present invention was completed.

Therefore, the present invention is directed to providing a method of providing information for diagnosing brain tumor through metagenomic analysis on bacteria-derived extracellular vesicles, a method of diagnosing brain tumor, a method of predicting the risk of the onset of brain tumor, and the like.

However, the technical goals of the present invention are not limited to the aforementioned goals, and other unmentioned technical goals will be clearly understood by those of ordinary skill in the art from the following description.

### [Technical Solution]

To achieve the above-described object of the present invention, the present invention provides a method of providing information for diagnosing brain tumor, comprising the following processes:
(a) extracting DNAs from extracellular vesicles isolated from a normal individual and a subject sample;
(b) performing polymerase chain reaction (PCR) on the extracted DNA using a pair of primers comprising SEQ ID NO: 1 and SEQ ID NO: 2; and
(c) comparing the increase or decrease in content of extracellular vesicles derived from bacteria of the subject-derived sample with that of the normal individual-derived sample through sequencing of the PCR product.

The present invention also provides a method of diagnosing brain tumor, comprising the following processes:
(a) extracting DNAs from extracellular vesicles isolated from a normal individual and a subject sample;
(b) performing PCR on the extracted DNA using a pair of primers comprising SEQ ID NO: 1 and SEQ ID NO: 2; and
(c) comparing the increase or decrease in content of extracellular vesicles derived from bacteria of the subject-derived sample with that of the normal individual-derived sample through sequencing of the PCR product.

The present invention also provides a method of predicting a risk of the onset of brain tumor, comprising the following processes:
(a) extracting DNAs from extracellular vesicles isolated from a normal individual and a subject sample;
(b) performing PCR on the extracted DNA using a pair of primers comprising SEQ ID NO: 1 and SEQ ID NO: 2; and
(c) comparing the increase or decrease in content of extracellular vesicles derived from bacteria of the subject-derived sample with that of the normal individual-derived sample through sequencing of the PCR product.

The present invention also provides a method of providing information for predicting the onset of brain tumor, comprising the following processes:
(a) extracting DNAs from extracellular vesicles isolated from a normal individual and a subject sample;
(b) performing PCR on the extracted DNA using a pair of primers comprising SEQ ID NO: 1 and SEQ ID NO: 2; and
(c) comparing the increase or decrease in content of extracellular vesicles derived from bacteria of the subject-derived sample with that of the normal individual-derived sample through sequencing of the PCR product.

In one embodiment of the present invention, in process (c), an increase or decrease in content of extracellular vesicles derived from one or more bacteria selected from the group consisting of the phylum *Actinobacteria,* the phylum *Proteobacteria,* the phylum *Firmicutes,* the phylum *Bacteroidetes,* the phylum *Cyanobacteria,* the phylum *Saccharibacteria,* and the phylum *Tenericutes* may be compared.

In another embodiment of the present invention, in process (c), an increase or decrease in content of extracellular vesicles derived from one or more bacteria selected from the group consisting of the class *Clostridia,* the class *Bacilli,* the class *Erysipelotrichiam,* the class *Gammaproteobacteria,* the class *Alphaproteobacteria,* the class *Negativicutes,* the class *Saccharibacteria (p),* the class *Chloroplast,* the class *Actinobacteria* and the class *Mollicutes* may be compared.

In another embodiment of the present invention, in process (c), an increase or decrease in content of extracellular vesicles derived from one or more bacteria selected from the group consisting of the order *Clostridiales,* the order *Bifidobacteriales,* the order *Erysipelotrichales,* the order *Lactobacillales,* the order *Micrococcales,* the order *Sphingomonadales,* the order *Selenomonadales,* the order *Chloroplast (c),* the order *Saccharibacteria (p),* the order *Turicibacterales* and the order *Xanthomonadales* may be compared.

In another embodiment of the present invention, in process (c), an increase or decrease in content of extracellular vesicles derived from one or more bacteria selected from the group consisting of the family *Ruminococcaceae,* the family *Peptostreptococcaceae,* the family *Erysipelotrichaceae,* the family *Lachnospiraceae,* the family *Streptococcaceae,* the family *Bifidobacteriaceae,* the family *Sphingomonadaceae,* the family *Porphyromonadaceae,* the family *Bacteroidaceae,* the family *Bacteroidales* S24-7, the family *Chloroplast (c),* the family *Prevotellaceae,* the family *Saccharibacteria (p),* the family *Intrasporangiaceae,* the family *Lactobacillaceae,* the family *Mogibacteriaceae,* the family *Turicibacteraceae,* the family *Nocardiaceae,* the family *Xanthomonadaceae,* the family *Weeksellaceae,* and the family *Veillonellaceae* may be compared.

In another embodiment of the present invention, in process (c), an increase or decrease in content of extracellular vesicles derived from one or more bacteria selected from the group consisting of the genus *Ruminococcaceae* UCG-014, the genus *Lachnospiraceae* NK4A136, the genus *Ruminococcaceae* UCG-013, the genus *Ruminiclostridium* 6, the genus *Peptoclostridium,* the genus *[Eubacterium] coprostanoligenes,* the genus *Escherichia-Shigella,* the genus *Blautia,* the genus *Bifidobacterium,* the genus *Streptococcus,* the genus *Bacteroides,* the genus *Erysipelatoclostridium,* the genus *Bacteroidales* S24-7, the genus *Chloroplast (c),* the genus *Prevotella* 9, the genus *Candidatus Saccharimonas,* the genus *Stenotrophomonas,* the genus *Lactobacillus,* the genus *Turicibacter,* the genus *Tepidimonas,* the genus *Sphingomonas,* the genus *Rhodococcus,* and the genus *Cloacibacterium* may be compared.

In another embodiment of the present invention, process (c) may comprise comparing an increase or decrease in content of extracellular vesicles derived from one or more bacteria selected from the group consisting of the phylum *Actinobacteria,* the phylum *Proteobacteria,* the phylum *Firmicutes,* the phylum *Bacteroidetes,* the phylum *Cyanobacteria,* the phylum *Saccharibacteria,* and the phylum *Tenericutes,*
extracellular vesicles derived from one or more bacteria selected from the group consisting of the class *Clostridia,* the class *Bacilli,* the class *Erysipelotrichiam,* the class *Gammaproteobacteria,* the class *Alphaproteobacteria,* the class *Negativicutes,* the class *Saccharibacteria (p),* the class *Chloroplast,* the class *Actinobacteria* and the class *Mollicutes,*
extracellular vesicles derived from one or more bacteria selected from the group consisting of the order *Clostridiales,* the order *Bifidobacteriales,* the order *Erysipelotrichales,* the order *Lactobacillales,* the order *Micrococcales,* the order *Sphingomonadales,* the order *Selenomonadales,* the order *Chloroplast (c),* the order *Saccharibacteria (p),* the order *Turicibacterales* and the order *Xanthomonadales,*
extracellular vesicles derived from one or more bacteria selected from the group consisting of the family *Ruminococcaceae,* the family *Peptostreptococcaceae,* the family *Erysipelotrichaceae,* the family *Lachnospiraceae,* the family *Streptococcaceae,* the family *Bifidobacteriaceae,* the family *Sphingomonadaceae,* the family *Porphyromonadaceae,* the family *Bacteroidaceae,* the family *Bacteroidales* S24-7, the family *Chloroplast (c),* the family *Prevotellaceae,* the family *Saccharibacteria (p),* the family *Intrasporangiaceae,* the family *Lactobacillaceae,* the family *Mogibacteriaceae,* the family *Turicibacteraceae,* the family *Nocardiaceae,* the family *Xanthomonadaceae,* the family *Weeksellaceae,* and the family *Veillonellaceae,* or
extracellular vesicles derived from one or more bacteria selected from the group consisting of the genus *Ruminococcaceae* UCG-014, the genus *Lachnospiraceae* NK4A136, the genus *Ruminococcaceae* UCG-013, the genus *Ruminiclostridium* 6, the genus *Peptoclostridium,* the genus *[Eubacterium] coprostanoligenes,* the genus *Escherichia-Shigella,* the genus *Blautia,* the genus *Bifidobacterium,* the genus *Streptococcus,* the genus *Bacteroides,* the genus *Erysipelatoclostridium,* the genus *Bacteroidales* S24-7, the genus *Chloroplast (c),* the genus *Prevotella* 9, the genus *Candidatus Saccharimonas,* the genus *Stenotrophomonas,* the genus *Lactobacillus,* the genus *Turicibacter,* the genus *Tepidimonas,* the genus *Sphingomonas,* the genus *Rhodococcus,* and the genus *Cloacibacterium.*

In another embodiment of the present invention, in process (c), in comparison with the normal individual-derived sample, it is possible to diagnose an increase in the content of the following as brain tumor:
extracellular vesicles derived from bacteria of the phylum *Tenericutes;*
extracellular vesicles derived from bacteria of the class *Mollicutes,*
extracellular vesicles derived from bacteria of the order *Turicibacterales,*
extracellular vesicles derived from one or more bacteria selected from the group consisting of the family *Lactobacillaceae,* the family *Mogibacteriaceae,* the family *Turicibacteraceae,* the family *Nocardiaceae,* and the family *Weeksellaceae,* or
extracellular vesicles derived from one or more bacteria selected from the group consisting of the genus *Lactobacillus,* the genus *Turicibacter,* the genus *Tepidimonas,* the genus *Rhodococcus,* and the genus *Cloacibacterium.*

In another embodiment of the present invention, in process (c), in comparison with the normal individual-derived sample, it is possible to diagnose a decrease in the content of the following as brain tumor:
extracellular vesicles derived from bacteria of the phylum *Actinobacteria,*
extracellular vesicles derived from bacteria of the class *Actinobacteria,*
extracellular vesicles derived from bacteria of the order *Xanthomonadales,*
extracellular vesicles derived from one or more bacteria selected from the group consisting of the family *Intrasporangiaceae,* the family *Xanthomonadaceae,* and the family *Veillonellaceae,* or
extracellular vesicles derived from one or more bacteria selected from the group consisting of the genus *Stenotrophomonas* and the genus *Sphingomonas.*

In another embodiment of the present invention, in process (c), in comparison with the normal individual-derived sample, it is possible to diagnose an increase in the content of the following as brain tumor:
extracellular vesicles derived from bacteria of the phylum *Firmicutes,*
extracellular vesicles derived from one or more bacteria selected from the group consisting of the class *Clostridia,* the class *Bacilli,* and the class *Erysipelotrichiam,*
extracellular vesicles derived from one or more bacteria selected from the group consisting of the order *Clostridiales,* the order *Bifidobacteriales,* and the order *Erysipelotrichales,*
extracellular vesicles derived from one or more bacteria selected from the group consisting of the family *Ruminococcaceae,* the family *Lactobacillaceae,* the family *Peptostreptococcaceae,* and the family *Erysipelotrichaceae,* or
extracellular vesicles derived from one or more bacteria selected from the group consisting of the genus *Ruminococcaceae* UCG-014, the genus *Lachnospiraceae* NK4A136, the genus *Ruminococcaceae* UCG-013, the genus *Lactobacillus,* the genus *Ruminiclostridium* 6, and the genus *Peptoclostridium.*

In another embodiment of the present invention, in process (c), in comparison with the normal individual-derived sample, it is possible to diagnose a decrease in the content of the following as brain tumor:
extracellular vesicles derived from one or more bacteria selected from the group consisting of the phylum *Actinobacteria* and the phylum *Proteobacteria,*
extracellular vesicles derived from one or more bacteria selected from the group consisting of the class *Gammaproteobacteria,* the class *Actinobacteria,* the class *Alphaproteobacteria,* and the class *Negativicutes,*
extracellular vesicles derived from one or more bacteria selected from the group consisting of the order *Lactobacillales,* the order *Micrococcales,* the order *Sphingomonadales,* and the order *Selenomonadales,*
extracellular vesicles derived from one or more bacteria selected from the group consisting of the family *Lachnospiraceae,* the family *Streptococcaceae,* the family *Bifidobacteriaceae,* the family *Sphingomonadaceae,* and the family *Porphyromonadaceae,* or
extracellular vesicles derived from one or more bacteria selected from the group consisting of the genus *[Eubacterium] coprostanoligenes,* the genus *Escherichia-Shigella,* the genus *Blautia,* the genus *Bifidobacterium,* the genus *Streptococcus,* and the genus *Sphingomonas.*

In another embodiment of the present invention, in process (c), in comparison with the normal individual-derived sample, it is possible to diagnose an increase in the content of the following as brain tumor:
extracellular vesicles derived from one or more bacteria selected from the group consisting of the phylum *Actinobacteria,* the phylum *Proteobacteria,* the phylum *Firmicutes,* and the phylum *Bacteroidetes,*
extracellular vesicles derived from bacteria of the class *Erysipelotrichiam,*
extracellular vesicles derived from bacteria of the order *Erysipelotrichales,*
extracellular vesicles derived from one or more bacteria selected from the group consisting of the family *Erysipelotrichaceae* and the family *Bacteroidaceae,* or
extracellular vesicles derived from one or more bacteria selected from the group consisting of the genus *Bacteroides* and the genus *Erysipelatoclostridium.*

In another embodiment of the present invention, in process (c), in comparison with the normal individual-derived sample, it is possible to diagnose a decrease in the content of the following as brain tumor:
extracellular vesicles derived from one or more bacteria selected from the group consisting of the phylum *Cyanobacteria* and the phylum *Saccharibacteria,*
extracellular vesicles derived from one or more bacteria selected from the group consisting of the class *Clostridia,* the class *Saccharibacteria (p),* and the class *Chloroplast,*
extracellular vesicles derived from one or more bacteria selected from the group consisting of the order *Clostridiales,* the order *Chloroplast (c),* and the order *Saccharibacteria (p)*,
extracellular vesicles derived from one or more bacteria selected from the group consisting of the family *Ruminococcaceae,* the family *Bacteroidales* S24-7, the family *Chloroplast (c),* the family *Prevotellaceae,* and the family *Saccharibacteria (p),* or
extracellular vesicles derived from one or more bacteria selected from the group consisting of the genus *Bacteroidales* S24-7, the genus *Chloroplast (c),* the genus *Lachnospiraceae* NK4A136, the genus *Prevotella* 9, and the genus *Candidatus Saccharimonas.*

In another embodiment of the present invention, the normal individual and subject sample may be blood or tissue.

In another embodiment of the present invention, the blood may be whole blood, serum, plasma, or blood mononuclear cells.

In another embodiment of the present invention, the tissue may be brain tissue.

### [Advantageous Effects]

Extracellular vesicles secreted from bacteria present in the environment are absorbed into the body to have a direct influence on inflammation, and it is difficult to diagnose a brain tumor early before symptoms appear, making effective treatment difficult. Therefore, by diagnosing the risk of the onset of a brain tumor through the metagenomic analysis of bacteria-derived extracellular vesicles using a human-derived sample according to the present invention, it is possible to diagnose and predict a risk group for a brain tumor early, and also to delay or prevent the onset of the disease through appropriate management. In addition, there are advantages that, even after the onset of a brain tumor, early diagnosis can be performed to reduce the incidence of the brain tumor, a therapeutic effect cannot be only increased, but also the disease can be improved and recurrence thereof can be prevented by avoiding exposure to causative factors through metagenomic analysis in patients diagnosed with a brain tumor.

### [Description of Drawings]

FIGS. 1A and 1B show (1A) alpha diversity (Chao1, Shannon and Simpson indices) and (1B) beta diversity of serum EV microbiome in a healthy control group (HC) and a brain tumor patient (BT) using PCoA based on Bray-Curtis similarity at the genus level.
FIGS. 2A to 2D show the difference in microbiome abundance between a health control group (HC) and a brain tumor patient (BT) in serum at the phylum and genus levels. FIG. 2A is a heatmap of abundance of serum microbial EV at the phylum level, FIG. 2B is a heatmap of abundance of serum microbial EVs at the genus level, FIG. 2C shows core microbial EVs at the phylum level, assessed using a t-test, and FIG. 2D shows them at the genus level.
FIGS. 3A and 3B show significantly different microbial EVs, determined using LEFSe analysis, at the phylum level (3A) and the genus level (3B).
FIGS. 4A to 4C show the abundance of microbiomes in serum at the class level (4A), the order level (4B), and the family level (4C).
FIGS. 5A and 5B show brain tumor diagnostic models based on serum EV microbiome at the genus level. FIG. 5A shows ROC curves for M1 (red), M2 (blue), M3 (green) and M4 (yellow), and FIG. 5B is an ROC curve for M5.
FIGS. 6A to 6E show the difference in microbiome abundance between a healthy control group (HC) and a brain tumor patient (BT) in brain tissue at the phylum and genus levels, in which FIGS. 6A and 6B are heatmaps of core microbial EV taxa in brain tissue at the phylum and genus levels, FIGS. 6C and 6D shows core microbial EVs assessed by t-test at the phylum and genus levels, and FIG. 6E shows significantly different microbial EV biomarkers determined by LEfSe analysis at the genus level.
FIGS. 7A to 7C show the abundance of microbiomes in brain tissue at the class level (7A), the order level (7B), and the family level (7C).
FIGS. 8A to 8D show a fold-change between a healthy control group (HC) and a brain tumor patient (BT) in serum and tissue at the phylum level (8A), the class level (8B), the order level (8C), and the family level (8D). Red indicates genera of microbial EVs significantly different between clinical groups in serum, green indicates genera of microbial EVs significantly different between clinical groups in brain tissue, and blue indicates genera of microbial EVs having a significant difference in both serum and tissue samples.
FIG. 9 shows a fold-change between a healthy control group (HC) and a brain tumor patient (BT) in serum and tissue at the genus level. Red indicates genera of microbial EVs significantly different between clinical groups in serum, green indicates genera of microbial EVs significantly different between clinical groups in brain tissue, and blue indicates genera of microbial EVs having a significant difference in both serum and tissue samples.
FIG. 10 shows the distribution of bacteria-derived EVs with significant diagnostic performance at the phylum level by metagenomic analysis after the bacteria-derived EVs are isolated from blood in a brain tumor patient and a normal individual.
FIG. 11 shows the distribution of bacteria-derived EVs with significant diagnostic performance at the class level by metagenomic analysis after the bacteria-derived EVs are isolated from blood in a brain tumor patient and a normal individual.
FIG. 12 shows the distribution of bacteria-derived EVs with significant diagnostic performance at the order level by metagenomic analysis after the bacteria-derived EVs are isolated from blood in a brain tumor patient and a normal individual.
FIG. 13 shows the distribution of bacteria-derived EVs with significant diagnostic performance at the family level by metagenomic analysis after the bacteria-derived EVs are isolated from blood in a brain tumor patient and a normal individual.
FIG. 14 shows the distribution of bacteria-derived EVs with significant diagnostic performance at the genus level by metagenomic analysis after the bacteria-derived EVs are isolated from blood in a brain tumor patient and a normal individual.

### [Best Mode]

The present invention relates to a method of diagnosing brain tumor through bacterial metagenomic analysis. The inventors of the present invention extracted genes from bacteria-derived extracellular vesicles using a normal individual and subject-derived sample, performed metagenomic analysis thereon, and identified bacteria-derived extracellular vesicles capable of acting as a causative factor of brain tumor.

Therefore, the present invention provides a method of providing information for diagnosing brain tumor, comprising the following processes:
(a) extracting DNAs from extracellular vesicles isolated from a normal individual and a subject sample;
(b) performing polymerase chain reaction (PCR) on the extracted DNA using a pair of primers comprising SEQ ID NO: 1 and SEQ ID NO: 2; and
(c) comparing the increase or decrease in content of extracellular vesicles derived from bacteria of the subject-derived sample with that of the normal individual-derived sample through sequencing of the PCR product.

The term "brain tumor diagnosis" as used herein refers to determining whether a patient has a risk for brain tumor, whether the risk for brain tumor is relatively high, or whether brain tumor has already occurred. The method of the present invention may be used to delay the onset of brain tumor through special and appropriate care for a specific patient, which is a patient having a high risk for brain tumor or prevent the onset of brain tumor. In addition, the method may be clinically used to determine treatment by selecting the most appropriate treatment method through early diagnosis of brain tumor.

The term "metagenome" as used herein refers to the total of genomes including all viruses, bacteria, fungi, and the like in isolated regions such as soil, the intestines of animals, and the like, and is mainly used as a concept of genomes that explains identification of many microorganisms at once using a sequencer to analyze non-cultured microorganisms. In particular, a metagenome does not refer to a genome of one species, but refers to a mixture of genomes, including genomes of all species of an environmental unit. This term originates from the view that, when defining one species in a process in which biology is advanced into omics, various species as well as existing one species functionally interact with each other to form a complete species. Technically, it is the subject of techniques that analyzes all DNAs and RNAs regardless of species using rapid sequencing to identify all species in one environment and verify interactions and metabolism. In the present invention, bacterial metagenomic analysis is performed using bacteria-derived extracellular vesicles isolated from, for example, serum.

In the present invention, the normal individual and subject sample may be blood, and the blood may be whole blood, serum, plasma, or blood mononuclear cells, but is not limited thereto.

In the present invention, the normal individual and subject sample may be tissue, and the tissue may be brain tissue, but is not limited thereto.

In an embodiment of the present invention, metagenomic analysis was conducted on the bacteria-derived extracellular vesicles, and then the analysis results were analyzed at phylum, class, order, family and genus levels, thereby identifying bacteria-derived vesicles, which can actually be a cause of the onset of brain tumor (see Table 8 in the present invention).

More specifically, in one embodiment of the present invention, as a result of bacterial metagenomic analysis on vesicles present in a serum sample from a subject, there were a significant difference between a brain tumor patient and a normal individual in the content of extracellular vesicles derived from bacteria, for example,
at the phylum level, the phylum *Firmicutes,* the phylum *Actinobacteria,* and the phylum *Proteobacteria,*
at the class level, the class *Clostridia,* the class *Bacilli,* the class *Erysipelotrichia,* the class *Gammaproteobacteria,* the class *Actinobacteria,* the class *Alphaproteobacteria,* and the class *Negativicutes,*
at the order level, the order *Clostridiales,* the order *Bifidobacteriales,* the order *Erysipelotrichales,* the order *Lactobacillales,* the order *Micrococcales,* the order *Sphingomonadales,* and the order *Selenomonadales,*
at the family level, the family *Ruminococcaceae,* the family *Lactobacillaceae,* the family *Peptostreptococcaceae,* the family *Erysipelotrichaceae,* the family *Lachnospiraceae,* the family *Streptococcaceae,* the family *Bifidobacteriaceae,* the family *Sphingomonadaceae,* and the family *Porphyromonadaceae,* and
at the genus level, the genus *Ruminococcaceae* UCG-014, the genus *Lachnospiraceae* NK4A136, the genus *Ruminococcaceae* UCG-013, the genus *Lactobacillus,* the genus *Ruminiclostridium* 6, the genus *Peptoclostridium,* the genus *[Eubacterium] coprostanoligenes,* the genus *Escherichia-Shigella,* the genus *Blautia,* the genus *Bifidobacterium,* the genus *Streptococcus,* and the genus *Sphingomonas* (see Example 3).

More specifically, in one embodiment of the present invention, as a result of bacterial metagenomic analysis on vesicles present in a brain tissue sample from a subject, there were a significant difference between a brain tumor patient and a normal individual in the content of extracellular vesicles derived from bacteria, for example,
at the phylum level, the phylum *Firmicutes,* the phylum *Bacteroidetes,* the phylum *Actinobacteria,* the phylum *Proteobacteria,* the phylum *Cyanobacteria,* and the phylum *Saccharibacteria,*
at the class level, the class *Erysipelotrichia,* the class *Clostridia,* the class *Saccharibacteria (p),* and the class *Chloroplast,*
at the order level, the order *Erysipelotrichales,* the order *Clostridiales,* the order *Chloroplast (c),* and the order *Saccharibacteria (p),*
at the family level, the family *Bacteroidaceae,* the family *Erysipelotrichaceae,* the family *Ruminococcaceae,* the family *Bacteroidales* S24-7 group, the family *Chloroplast (c),* the family *Prevotellaceae,* and the family *Saccharibacteria (p),* and
at the genus level, the genus *Bacteroides,* the genus *Erysipelatoclostridium,* the genus *Bacteroidales* S24-7 group, the genus *Chloroplast (c),* the genus *Lachnospiraceae* NK4A136 group, the genus *Prevotella* 9, and the genus *Candidatus Saccharimonas* (see Example 5).

More particularly, in one embodiment of the present invention, as a result of performing bacterial metagenomic analysis on vesicles present in subject-derived blood samples at a phylum level, the content of extracellular vesicles derived from bacteria of the phylum *Actinobacteria* and the phylum *Tenericutes* was significantly different between brain tumor patients and normal individuals (see Example 6).

More particularly, in one embodiment of the present invention, as a result of performing bacterial metagenomic analysis on vesicles present in subject-derived blood samples at a class level, the content of extracellular vesicles derived from bacteria of the class *Actinobacteria* and the class *Mollicutes* was significantly different between brain tumor patients and normal individuals (see Example 6).

More particularly, in one embodiment of the present invention, as a result of performing bacterial metagenomic analysis on vesicles present in subj ect-derived blood samples at an order level, the content of extracellular vesicles derived from bacteria of the order *Turicibacterales,* the order *Xanthomonadales,* and the order RF39 was significantly different between brain tumor patients and normal individuals (see Example 6).

More particularly, in one embodiment of the present invention, as a result of performing bacterial metagenomic analysis on vesicles present in subj ect-derived blood samples at a family level, the content of extracellular vesicles derived from bacteria of the family *Intrasporangiaceae,* the family *Lactobacillaceae,* the family *Mogibacteriaceae,* the family *Turicibacteraceae,* the family *Nocardiaceae,* the family *Xanthomonadaceae,* the family *Weeksellaceae,* and the family *Veillonellaceae* was significantly different between brain tumor patients and normal individuals (see Example 6).

More particularly, in one embodiment of the present invention, as a result of performing bacterial metagenomic analysis on vesicles present in subject-derived blood samples at a genus level, the content of extracellular vesicles derived from bacteria of the genus *Stenotrophomonas,* the genus *Lactobacillus,* the genus *Turicibacter,* the genus *Tepidimonas,* the genus *Sphingomonas,* the genus *Rhodococcus,* and the genus *Cloacibacterium* was significantly different between brain tumor patients and normal individuals (see Example 6).

Through the results of the examples, it was confirmed that distribution variables of the identified extracellular vesicles derived from bacteria could be usefully used for the prediction of the onset of brain tumor.

### [Modes of the Invention]

Hereinafter, the present invention will be described with reference to exemplary examples to aid in understanding of the present invention. However, these examples are provided only for illustrative purposes and are not intended to limit the scope of the present invention.

### [Examples]

### Experimental Example 1. Subjects and sample collection

Serum samples of a total of 182 brain tumor (BT) patients and 125 healthy control (HC) subjects were collected from Seoul National University Hospital and Inje University Haeundae Hospital, respectively. In addition, tissue samples of 5 BT patients and 5 HC subjects collected from Seoul National University Hospital were evaluated. Each BT clinical subject had symptoms making him/her visit a hospital for treatment. Healthy control subjects were screened through a general health examination. The experimental example of the present invention was approved by the Institutional Review Boards of Seoul National University Hospital (IRB No. H-1009-025-331) and Inje University Haeundae Hospital (IRB No. 1297992-2015-064). All methods of the present invention were conducted according to approved guidelines, and informed consent was obtained from all clinical subjects.

All collected human serum samples were transferred to serum separator tubes (SSTs), and then centrifuged at 4 °C for 15 minutes at 3,000 rpm. All brain tissue samples were frozen in liquid nitrogen and stored at -80 °C for analysis.

### Experimental Example 2. In vivo mouse research model

All mice used herein were 6-week-old female C57BL/6 mice (Orient Bio Inc., Seongnam, Korea). The mice were housed and maintained under standard laboratory conditions of 22±2 °C and 50±5% humidity at 12-hour day/night cycles throughout the *in vivo* research.

### Experimental Example 3. EV DNA extraction and sequencing

To extract EVs from serum and tissue samples, blood was put into a 10-mL tube, centrifuged (3,500 x g, 10 min, 4 °C) to settle the floating matter, followed by recovery of only a supernatant and transfer into a novel 10 mL tube. Bacteria and impurities were removed from the recovered supernatant using a 0.22-µm filter, transferred to centrifugal filters (50 kD) and centrifuged at 1500 x g and 4 °C for 15 minutes, and then substances smaller than 50 kD were discarded and concentrated up to 10 mL. Again, bacteria and impurities were removed using a 0.22-µm filter, the resulting supernatant was removed through ultracentrifugation using a Type 90ti rotor at 150,000 x g and 4 °C for 3 hours, and the lumpy pellet was suspended with PBS, thereby obtaining EVs.

100 µl of the vesicles isolated from the blood according to the above-described method was boiled at 100 °C to allow the internal DNA to come out of the lipid and then cooled on ice for 5 mins. Next, the resulting vesicles were centrifuged at 10,000 x g and 4 °C for 30 minutes to remove the remaining suspension, only the supernatant was collected, and then the amount of DNA extracted was quantified using a NanoDrop. In addition, to verify whether bacteria-derived DNA was present in the extracted DNA, PCR was performed using 16s rDNA primers shown in Table 1 below, and it was confirmed bacteria-derived DNA was present in the extracted DNA.

**[Table 1]**

| Primer | | Sequence | SEQ ID NO. |
|---|---|---|---|
| 16S rDNA | 16S_V3_F | 5'- TCGTCGGCAGCGTCAGATG TGTATAAGAGACAGCCTAC GGGNGGCWGCAG-3' | 1 |
| | 16S_V4_R | 5'- GTCTCGTGGGCTCGGAGAT GTGTATAAGAGACAGGACT ACHVGGGTATCTAATCC-3' | 2 |

### Experimental Example 4. Metagenomic analysis of microbial EV composition

Genes were extracted by the method of Experimental Example 3, amplified by PCR using the 16S rDNA primers shown in Table 1, and subjected to sequencing (Illumina MiSeq sequencer). Taxonomic assignment was performed by the profiling program MDx-Pro ver.2 (MD Healthcare, Korea). Paired-end reads were filtered according to barcodes, primer sequences were trimmed using Cutadapt (version 1.1.6), and merged with CASPER. To obtain high-quality sequencing reads, reads of less than 350 bp or more than 550 bp, and Phred quality scores of lower than 20 were excluded. Based on a 97% similarity threshold, operational taxonomic units (OTUs) were assigned at the genus level using the VSEARCH de novo clustering method. OTUs including one sequence in only one sample were excluded. Subsequently, taxonomic assignment was performed at the species level using UCLUST and QIIME 1.9.1 for the Silva 132 database under default parameters. When clusters could be assigned at the genus level due to insufficient taxonomic information in the database, the taxon was assigned the next highest level. Parentheses around the taxon name mainly indicate the proposed taxonomic assignment, which has not been identified, based on whole genome phylogeny in the genomic database.

### Experimental Example 5. Establishment of predictive diagnostic model

To develop a BT predictive diagnostic model, the relative abundance of OTUs at the genus level was considered as a model variable.

First, candidate strains which have a p value of less than 0.01, a fold-change higher than 2-fold, and an average relative abundance of more than 0.1% were selected. The selected strains as model variables were compared to determine a model with the highest Area Under the Curve (AUC), sensitivity, specificity and accuracy.

The first method (M1) used stepwise selection in which the Akaike information criterion (AIC) is used to compare predictive diagnostic models with different variables. The second method (M2) incorporated age and sex as covariates in addition to the stepwise selection methodology. The third method (M3) used linear discriminant analysis (LDA) and LDA effect size (LEfSe) algorithms to find strain markers. The fourth method (M4) included age and sex as covariates in addition to the incorporation of the selected strain markers using LEfSe. In addition, the fifth method (M5) was calculated by a machine learning algorithm based on the gradient boosting machine (GBM) ensemble method.

GBM was incorporated in modeling using the gradient boosting regressor (version 0.21.3) of scikit-learn in python (version 3.6.9). After variable selection, the predictive diagnostic model was calculated using logistic regression with training and a test set in a 80:20 ratio for model validation.

### Experimental Example 6. Statistical analysis

Significant age differences between the BT and the control were determined through the Student's t-test and the Wilcoxon rank-sum test, respectively. To determine a statistical difference between groups based on the sex of a cohort, the Chi-square test was performed. Praycipal coordinate analysis (PCoA) was performed to determine individual taxa-level clusterings of groups based on Bray-Curtis dissimilarity. To analyze the difference in microbiome composition between the HC group and the BT group, the Student's t-test was performed. LEfSe was used to determine significant, differentially abundant genera between clinical groups to select biomarkers with statistical and biological significance. The LEfSe algorithm used the Wilcoxon rank-sum test and linear discriminate analysis (LDA) to set the cut-off LDA score (log10) to 2. When the p value was less than 0.05 (p<0.05), the results were considered significant, and all analyses were performed using R version 3.6.1.

### Example 1. Clinical characteristics

Through evaluation of the clinical characteristics of HC and BT subject groups, it was determined that there was a significant difference between the two groups (p <0.001). The HC subjects ranged from 40 to 78 years old, their average age was 59.7 (SD 10.5), the BT subjects ranged from 16 to 81 years old, and their average age was 51.5 (SD 14.2) (Table 2).

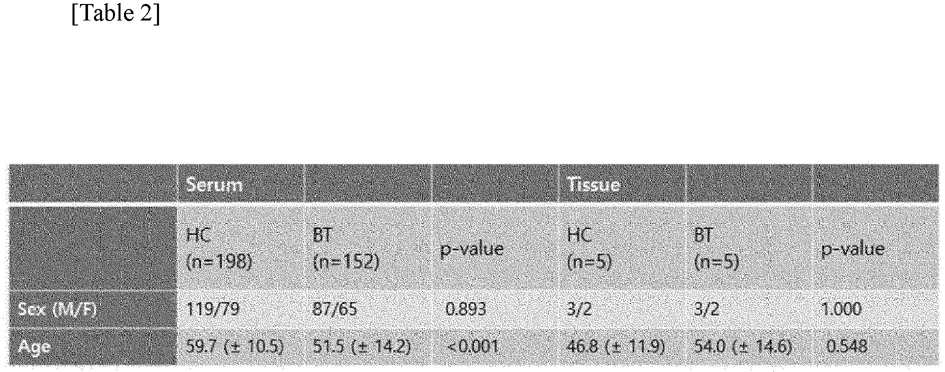

### Example 2. Diversity

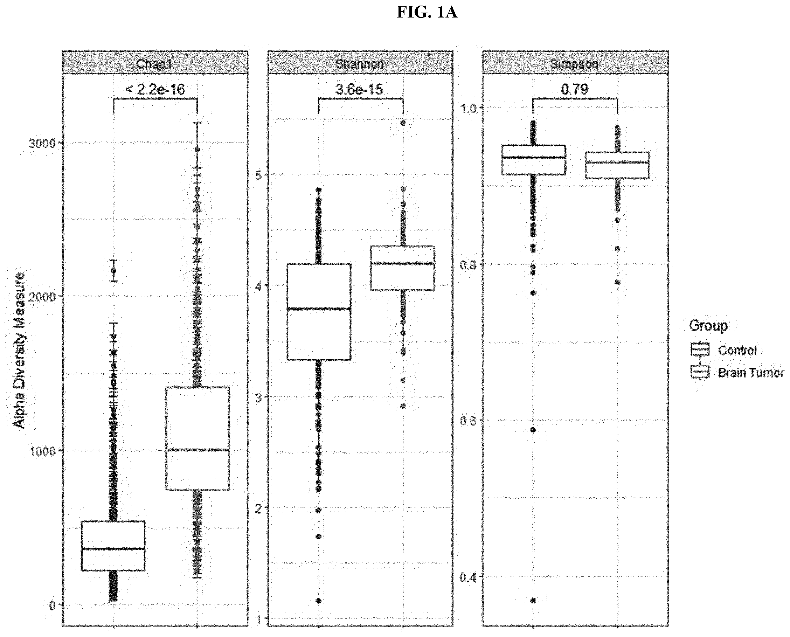

The Chao1 index of species richness and Shannon index of bacterial clustering diversity were significantly high in BT group, but the difference in Simpson index between the BT and control groups was not significant (FIG. 1A).

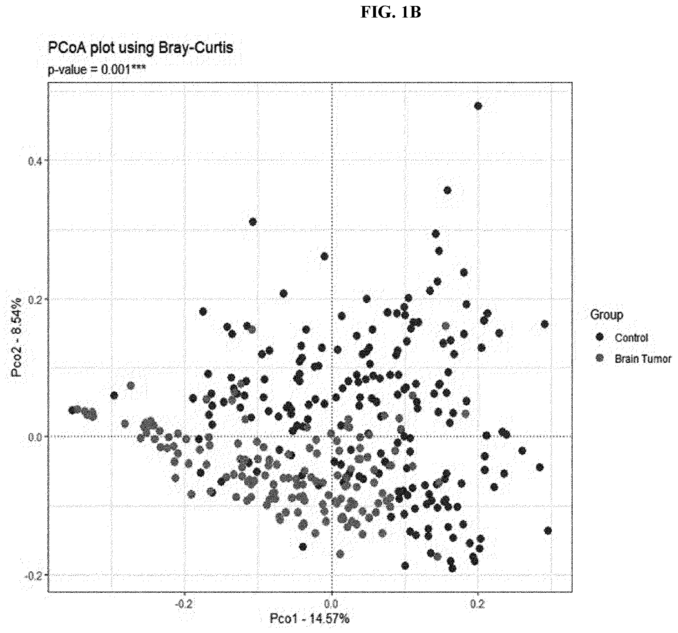

PCoA was performed, and all samples were plotted along two principal coordinates (PCos) which are most dissimilar between samples to evaluate the similarity between HC and BT groups. At all taxa levels, significant clustering was observed between two groups (p <0.001) (FIG. 1B).

### Example 3. Confirmation of microbial EV abundance in serum and biomarkers

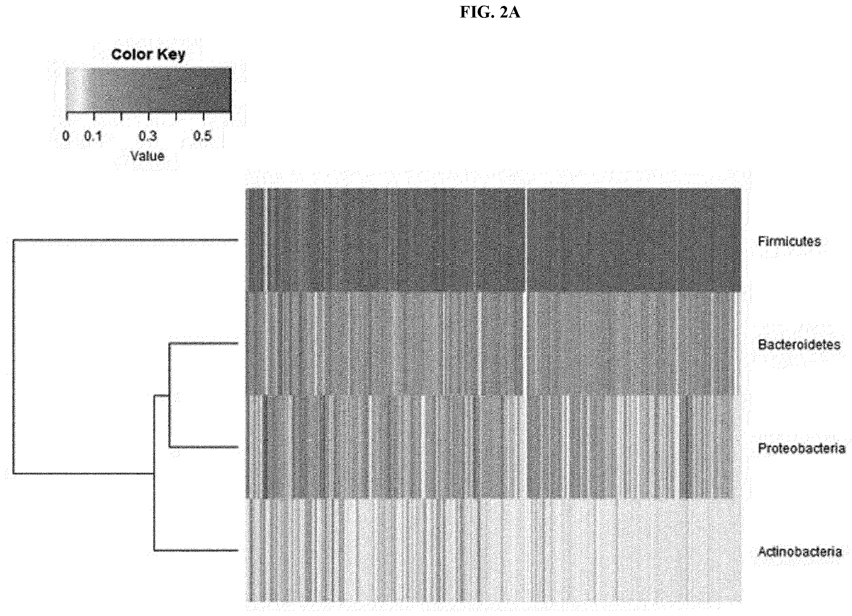

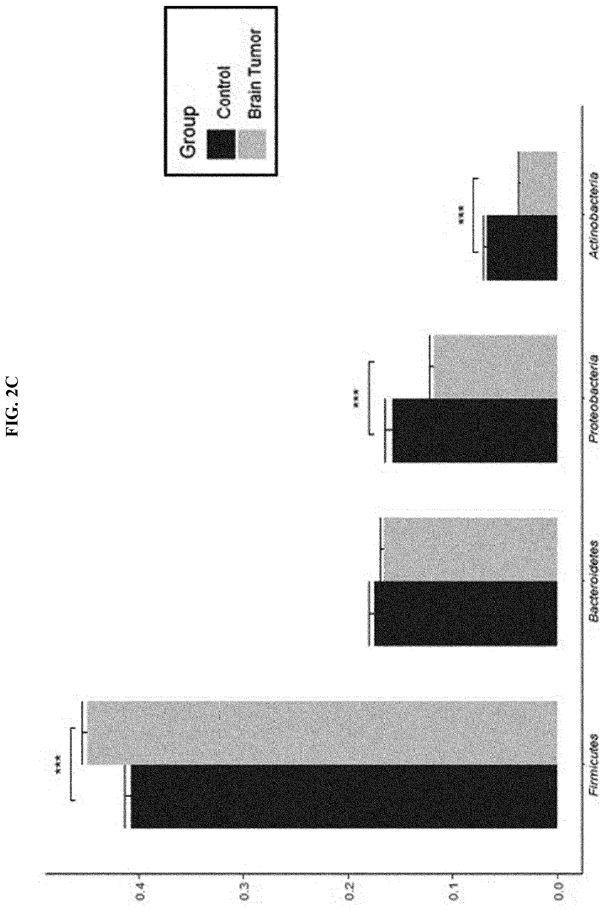

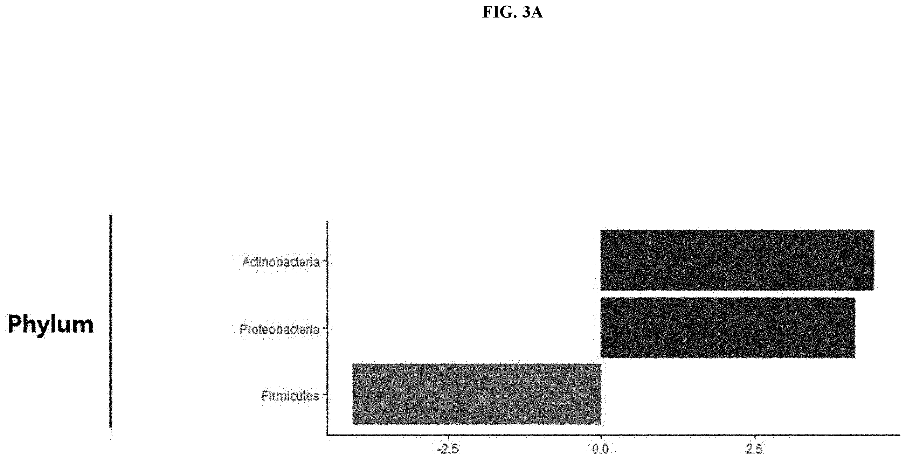

At the phylum level, *Firmicutes* abundance was significantly lower, but *Actinobacteria* and *Proteobacteria* were higher in the control than in the patient group (FIGS. 2A and 2C). LEfSe analysis for phylum-level biomarkers deduced *Actinobacteria, Proteobacteria* and *Firmicutes* as the only phyla with a log (LDA score) value higher than 4 (FIG. 3A).

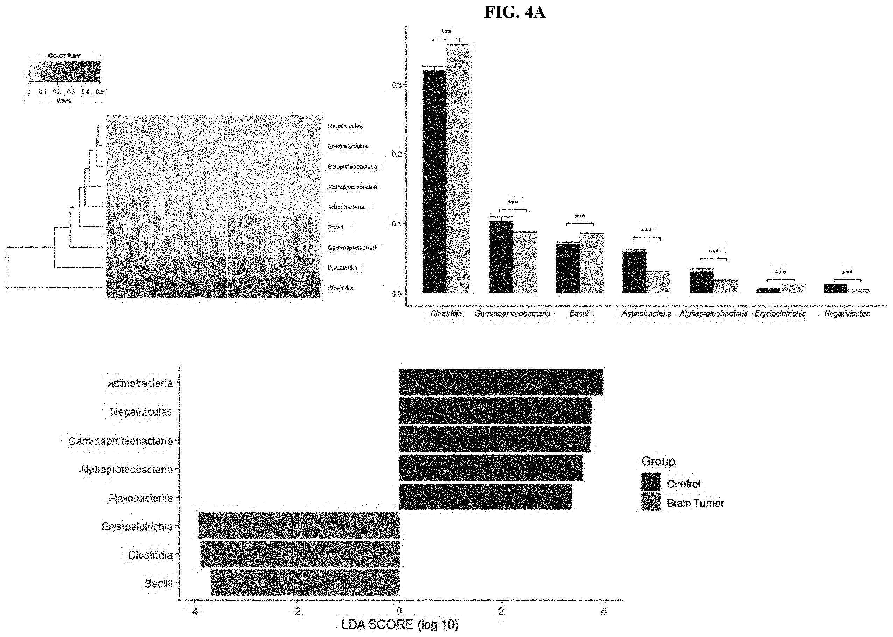

At the class level, *Clostridia, Bacilli, Erysipelotrichia, Gammaproteobacteria, Actinobacteria, Alphaproteobacteria* and *Negativicutes* were significantly changed. Eight class-level biomarkers were determined using *Actinobacteria* yielding a log (LDA score) of 4.0, indicating biologically significant higher abundance in the control (FIG. 4A).

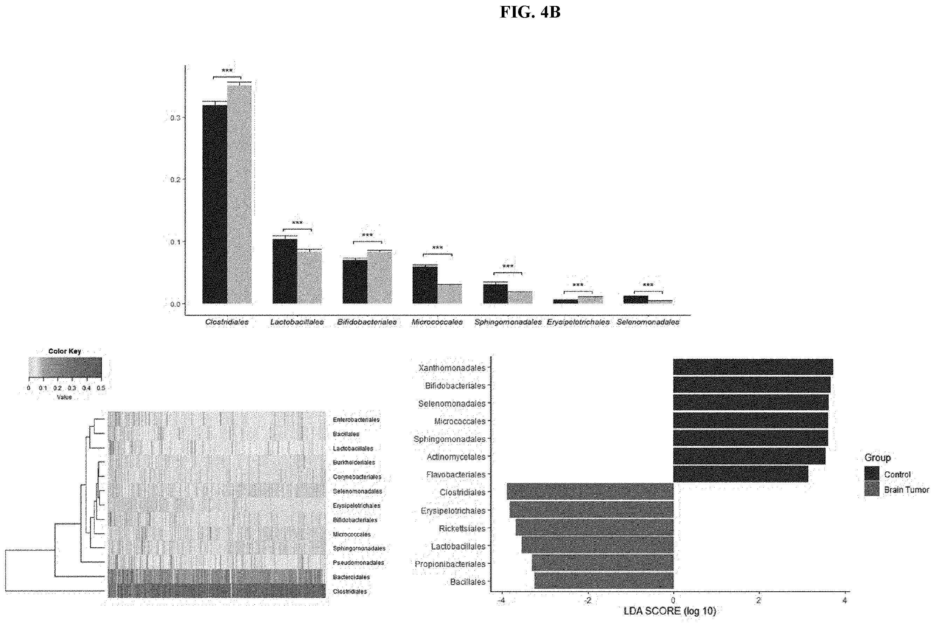

At the order level, *Clostridiales, Bifidobacteriales, Erysipelotrichales, Lactobacillales, Micrococcales, Sphingomonadales* and *Selenomonadales* were significantly changed between the control and the patient group. At the order level, as a result of LEfSe evaluation, 13 taxa were significantly different between the group and the patient group, and *Clostridiales* showed the highest significance with a log (LDA score) of 3.9 (FIG. 4B).

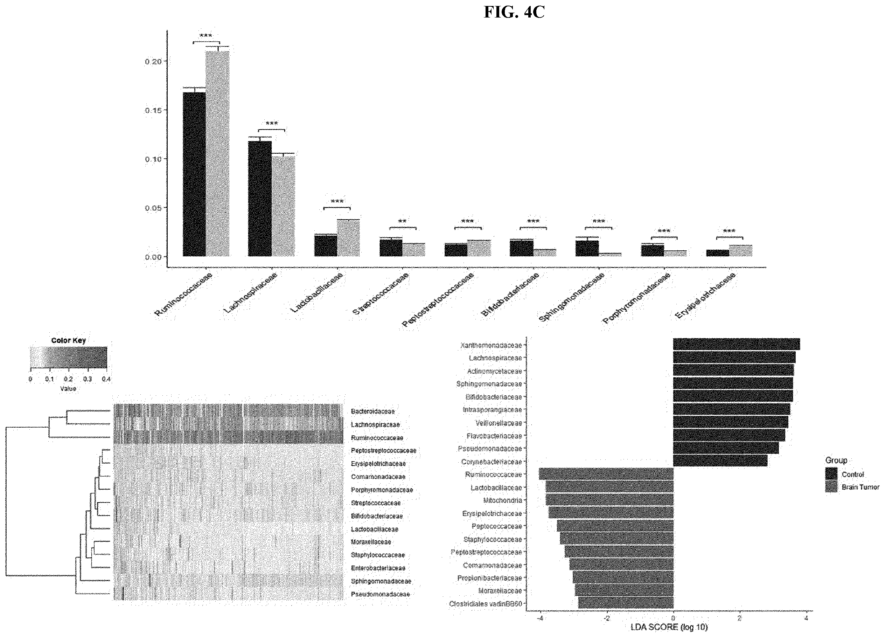

At the family level, *Ruminococcaceae, Lactobacillaceae, Peptostreptococcaceae, Erysipelotrichaceae, Lachnospiraceae, Streptococcaceae, Sphingomonadaceae* and *Porphyromonadaceae* were greatly changed. At the family level, as a result of LEfSe evaluation, a total of 21 taxa were significantly changed, and *Ruminococcaceae* had a log (LDA score) of more than 4.0 (FIG. 4C).

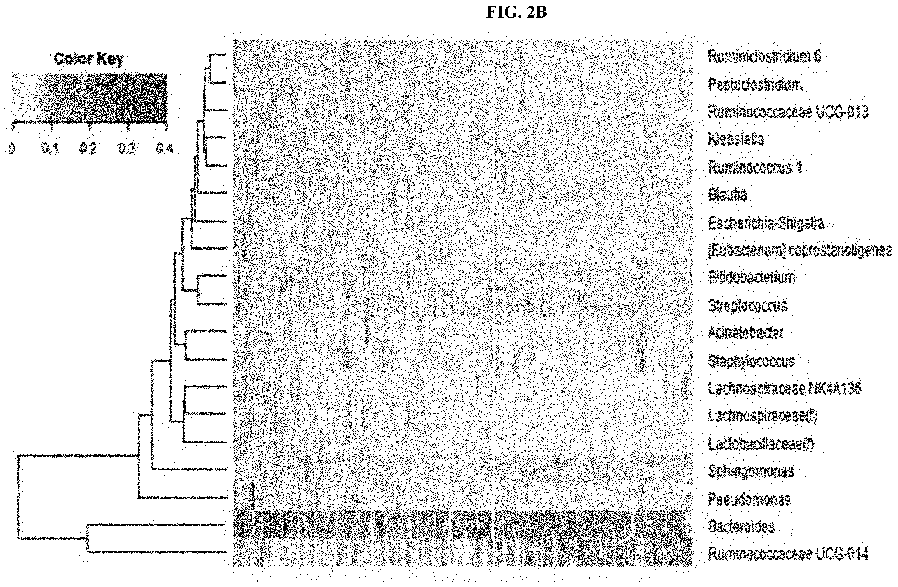

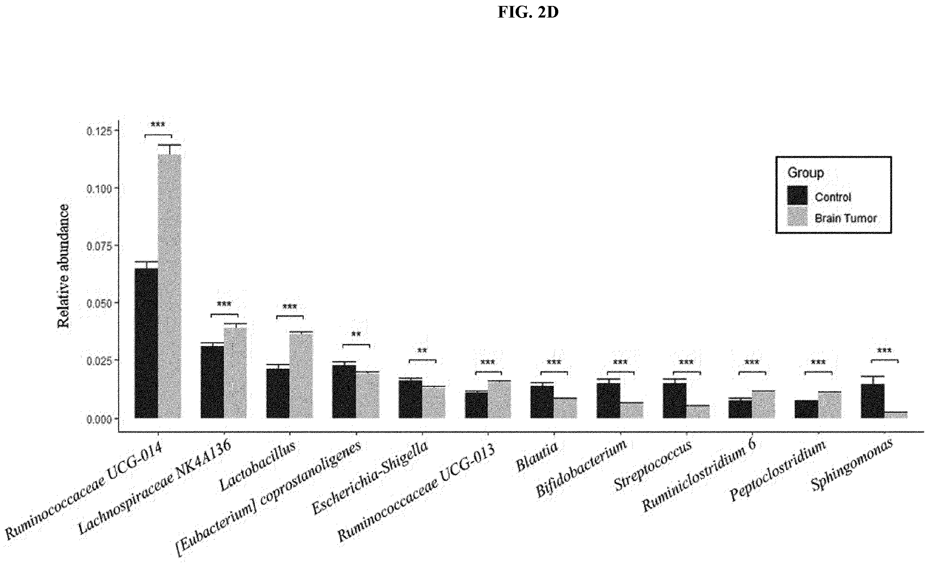

Finally, as a result of genus-level analysis, various taxa showing significant differences between the control and the BT group were determined (FIG. 2B). *Ruminococcaceae* UCG-014, *Lachnospiraceae* NK4A136, *Ruminococcaceae* UCG-013, *Lactobacillus, Ruminiclostridium* 6 and *Peptoclostridium* were significantly higher in the BT group than the control, whereas *[Eubacterium] coprostanoligenes, Escherichia-Shigella, Blautia, Bifidobacterium, Streptococcus* and *Sphingomonas* were significantly lower (FIG. 2D).

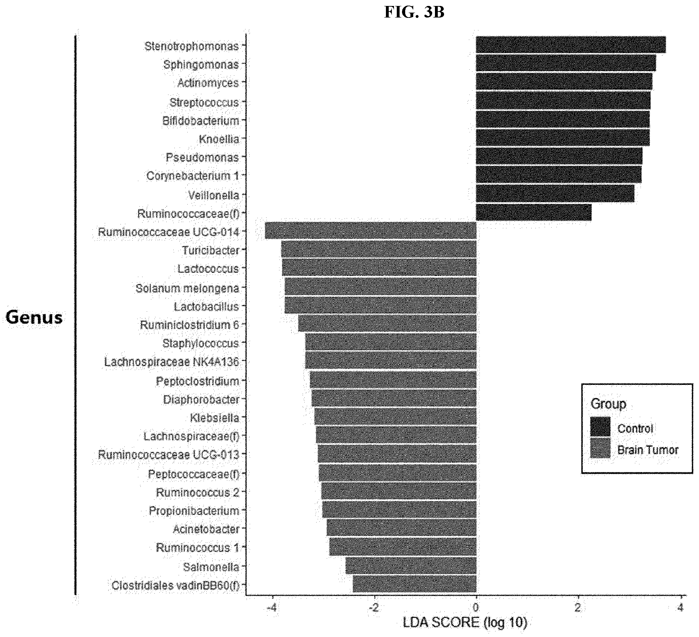

LEfSe analysis of a genus-level serum EV microbiome composition yielded a total of 30 genera, and particularly, it was shown that *Ruminococcaceae* UCG-014 has a log of more than 4.0 (FIG. 3B). A total of 4, 9, 12, 18 and 29 taxa showed a proportion higher than 0.5% in all groups, and a significant difference between the control and the patient group using a t-test at the phylum, class, order, family and genus levels (p <0.05).

### Example 4. Development of BT diagnostic model based on serum microbial EV metagenome

Using the serum microbial EV metagenomic profiles of the control and the BT groups, diagnostic models were developed to determine the risk of brain tumors in healthy subjects.

Following stepwise selection and logistic regression analysis, M1 and M2 models yielded 12 significant microbial EV genera, such as *Stenotrophomonas, Knoellia, Sphingomonas, Solanum melongena, Parabacteroides, Actinomyces, Ruminiclostridium, Lactococcus, Turicibacter, Faecalibacterium, Streptococcus,* and *Bifidobacterium.*

Meanwhile, logistic regression analysis using biomarkers determined by LEfSe analysis for M3 and M4 revealed 29 different significant microbial EV genera, such as *Ruminococcaceae* UCG-014, *Lachnospiraceae* NK4A136, *Lactobacillus, Lachnospiraceae(f), Acinetobacter, Staphylococcus, Pseudomonas, Ruminococcaceae* UCG-013, *Klebsiella, Bifidobacterium, Ruminococcus 1, Streptococcus, Ruminiclostridium* 6, *Peptoclostridium, Sphingomonas, Clostridiales vadinBB60(f), Turicibacter, Ruminococcaceae(f), Ruminococcus 2, Peptococcaceae(f), Diaphorobacter, Corynebacterium* 1, *Lactococcus, Propionibacterium, Solanum melongena, Actinomyces, Knoellia, Stenotrophomonas,* and *Veillonella.*

In the case of M5, the relative abundance of the analyzed total microbial EV metagenomic information was input as characteristics for analysis, rather than specific biomarkers.

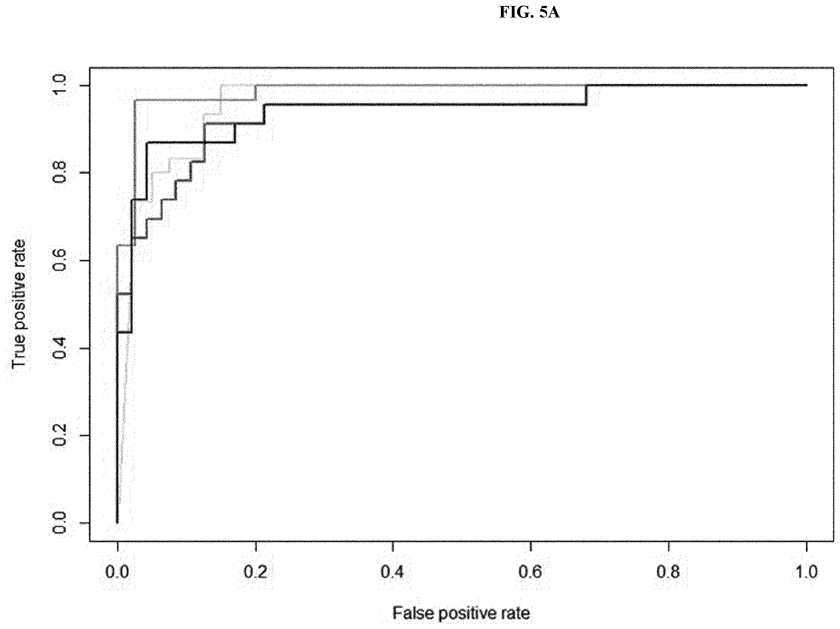

Model performance using the test set was evaluated based on the AUC, sensitivity, specificity and accuracy of each method to determine the optimal BT diagnostic model. As a result, all of the BT diagnostic models had an AUC higher than 0.93 (FIG. 5A).

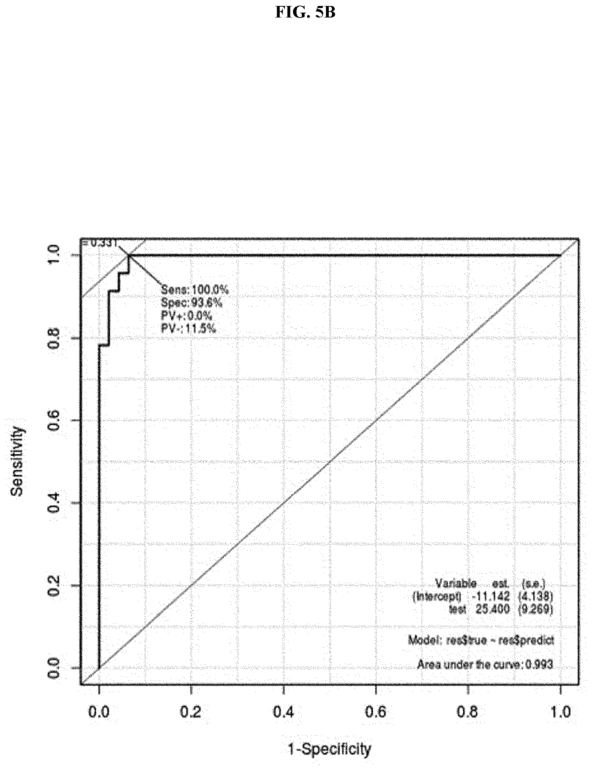

The model based on GBM had the highest sensitivity, specificity and AUC of 1.000, 0.936 and 0.993, respectively (FIG. 5B).

### Example 5. Development of BT diagnostic model based on microbial EV metagenome in brain tissue

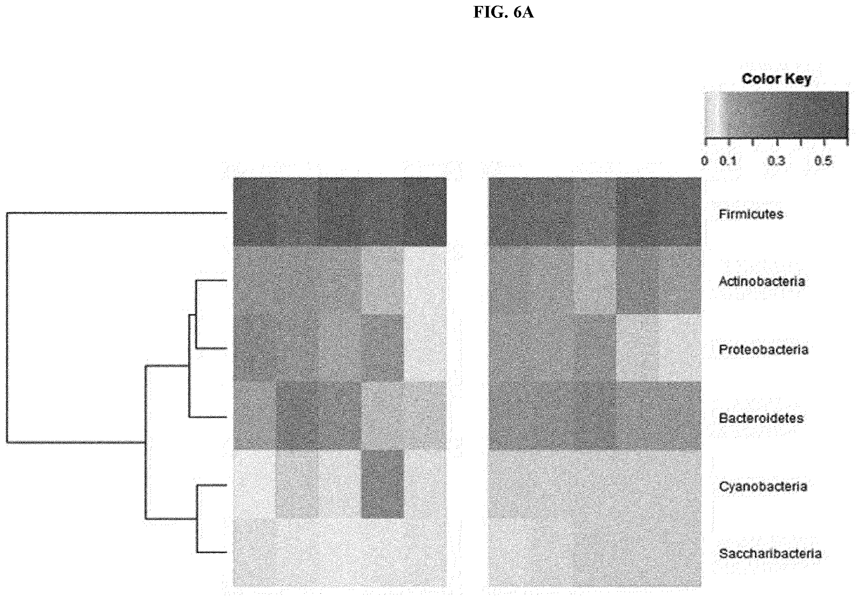

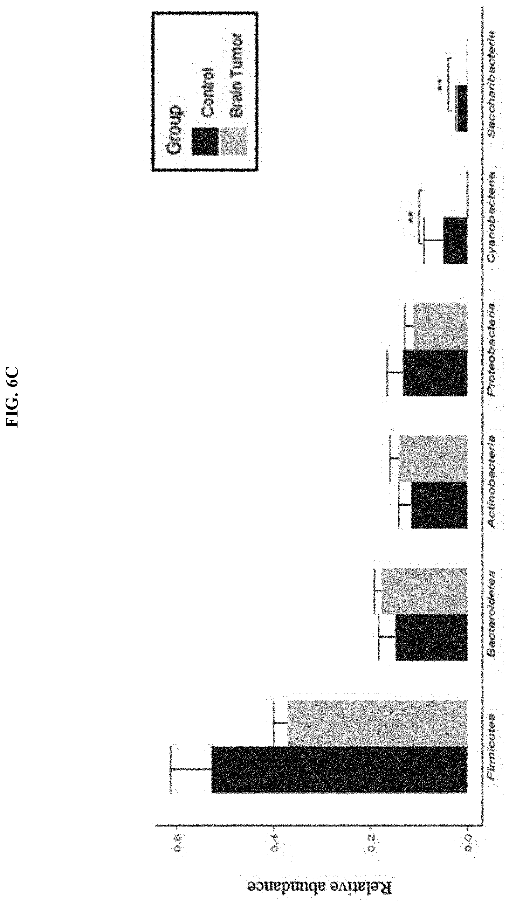

At the phylum level, *Firmicutes, Bacteroidetes, Actinobacteria* and *Proteobacteria* were the most abundant phyla in all groups, and in the patient group and control, accounted for 80% or more of the tissue EV taxa. *Cyanobacteria* and *Saccharibacteria* were significantly higher than in the control than the patient group (FIGS. 6A and 6C).

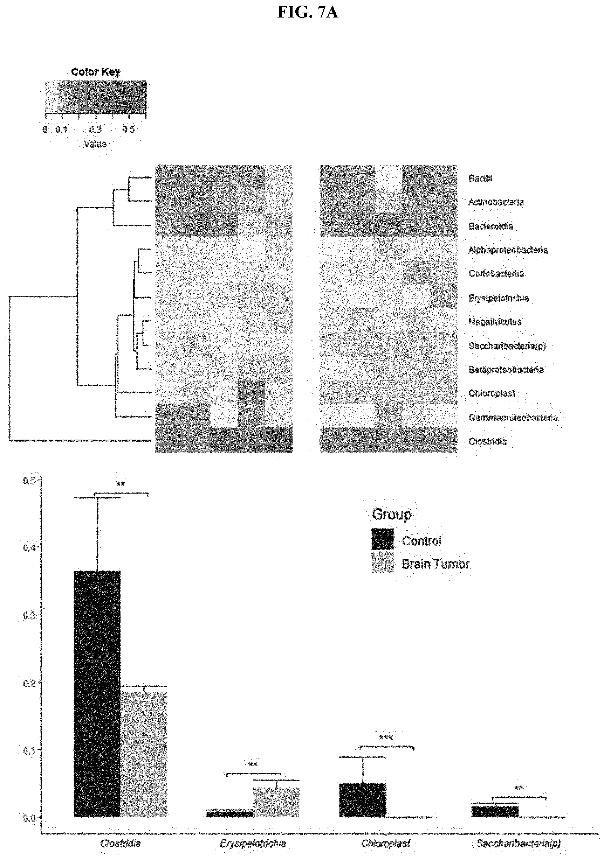

At the class level, *Erysipelotrichia* was significantly lower in the control than the patient group. However, *Clostridia, Saccharibacteria (p)* and *Chloroplast* were significantly higher in the control (FIG. 7A).

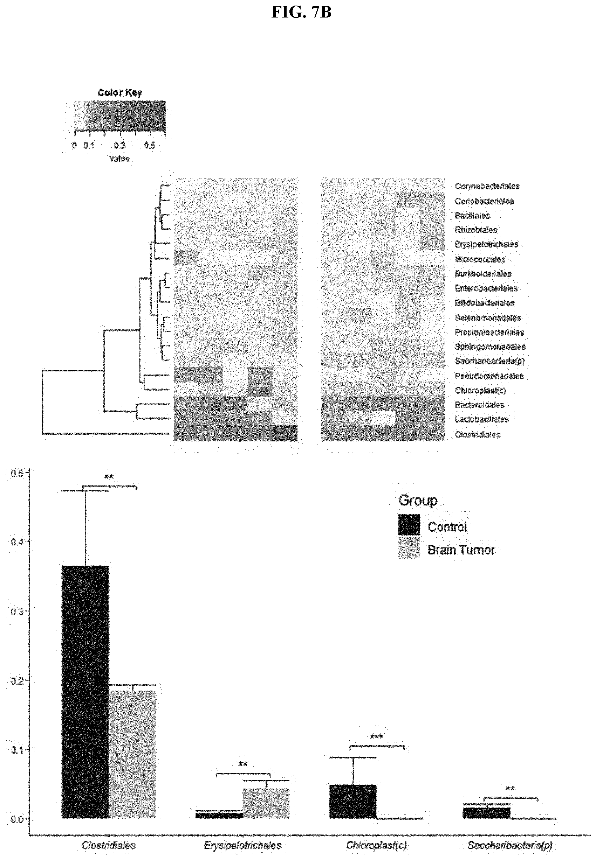

At the order level, *Clostridiales, Chloroplast (c)* and *Saccharibacteria (p)* were significantly abundant in the control, whereas *Erysipelotrichales* was significantly lower (FIG. 7B).

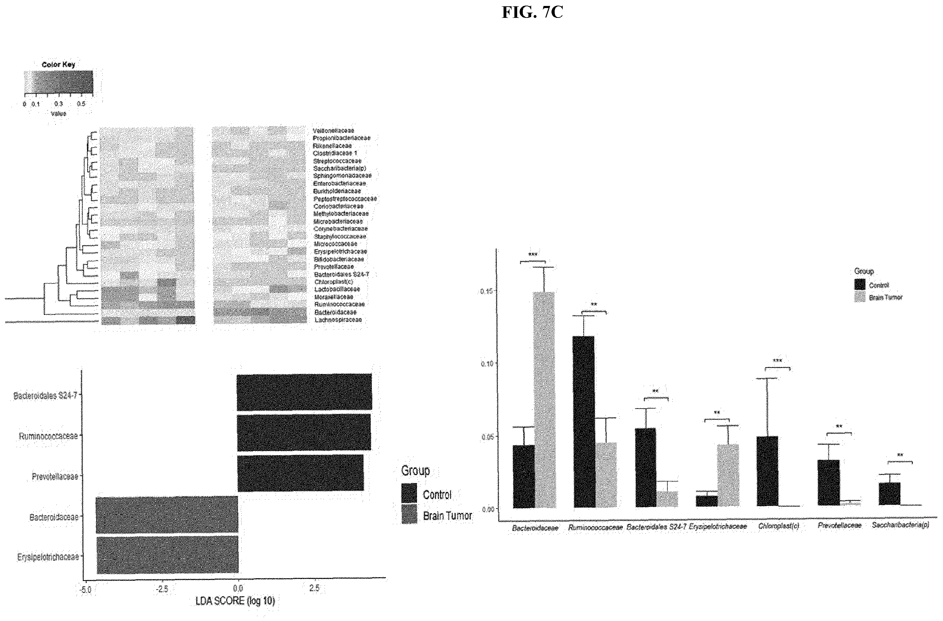

At the family level, *Bacteroidaceae, Ruminococcaceae,* the *Bacteroidales* S24-7 group, *Erysipelotrichaceae, Chloroplast (c), Prevotellaceae* and *Saccharibacteria (p)* were significantly changed between clinical groups, in the biomarkers found by LEfSe, the *Bacteroidales* S24-7 group, *Ruminococcaceae, Prevotellaceae, Bacteroidaceae* and *Erysipelotrichaceae* were included (FIG. 7C).

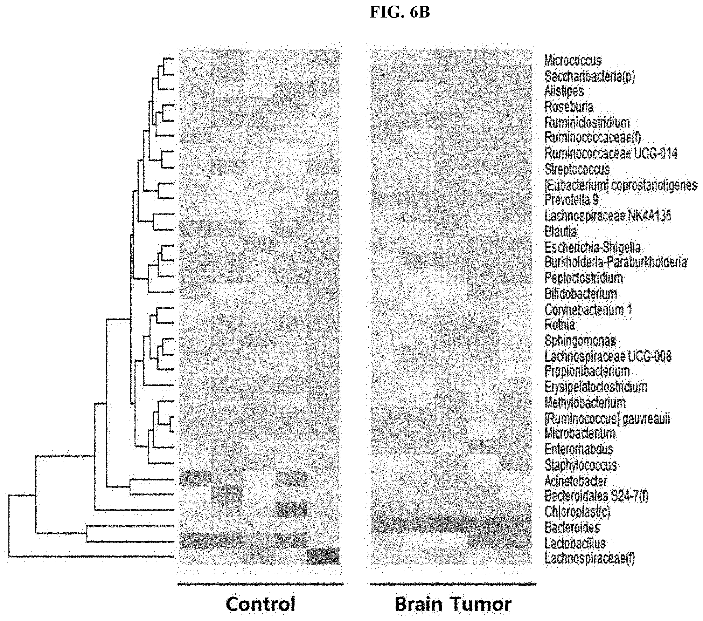

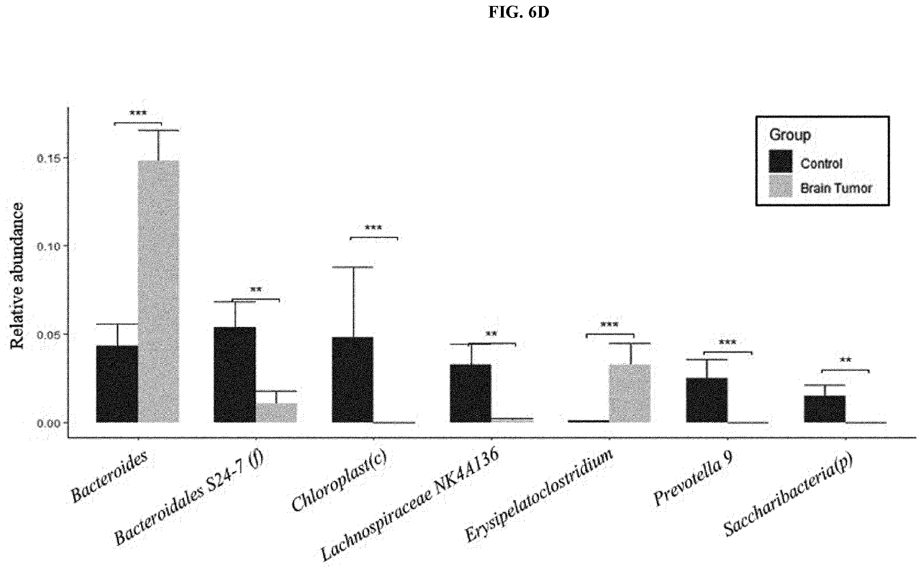

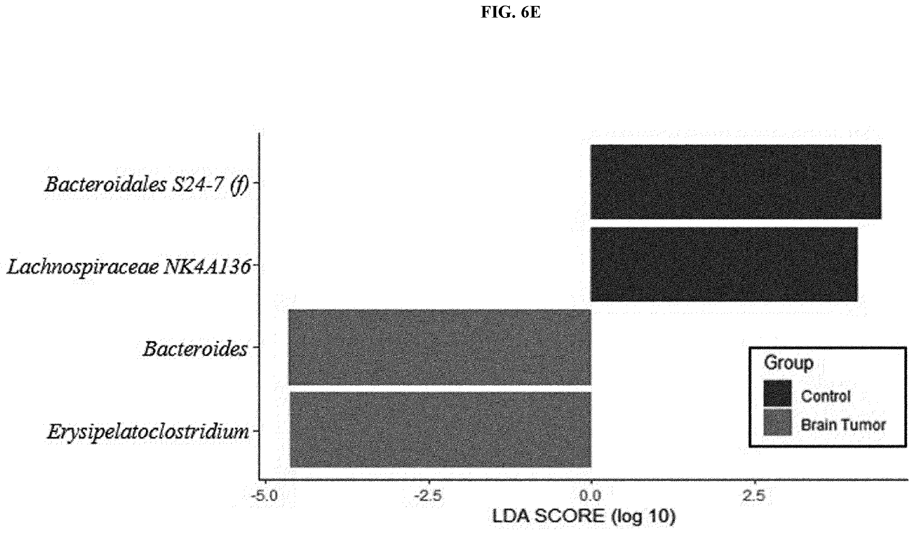

At the genus level, *Bacteroides* and *Erysipelatoclostridium* were significantly lower, whereas the *Bacteroidales* S24-7 group, *Chloroplast (c),* the *Lachnospiraceae* NK4A136 group, *Prevotella* 9, and *Candidatus Saccharimonas* were significantly higher in the control than in the BT group (FIGS. 6B and 6D). At the genus level, LEfSe evaluation showed the *Bacteroidales* S24-7 group, *Lachnospiraceae* NK4A136, *Bacteroides* and *Erysipelatoclostridium* as important BT biomarkers (FIG. 6E).

In addition, to compare the microbiome composition changes in serum and tissue between the control and the patients, microbial EV compositions in serum and tissue obtained from the same individual were analyzed.

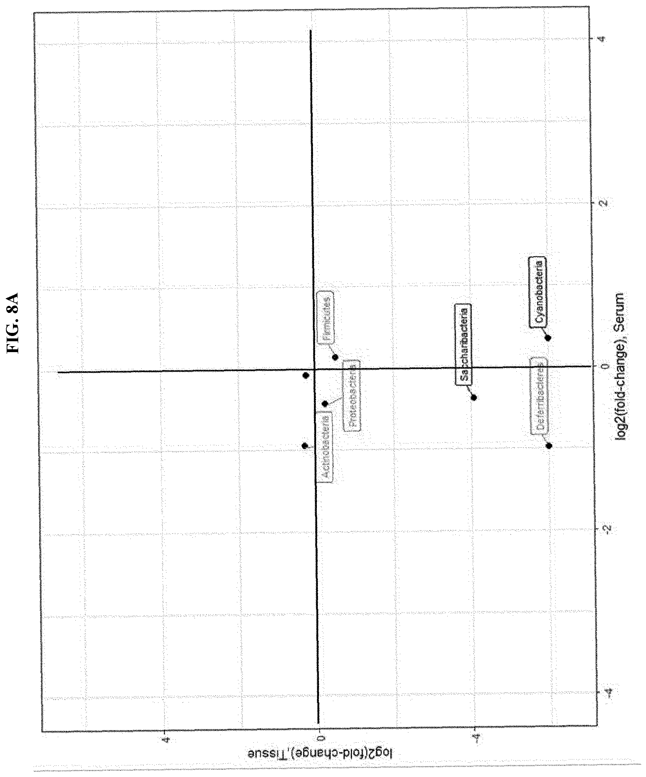

At the phylum level, *Saccaribacteria* in a patient was significantly decreased in both serum and tissue (FIG. 8A).

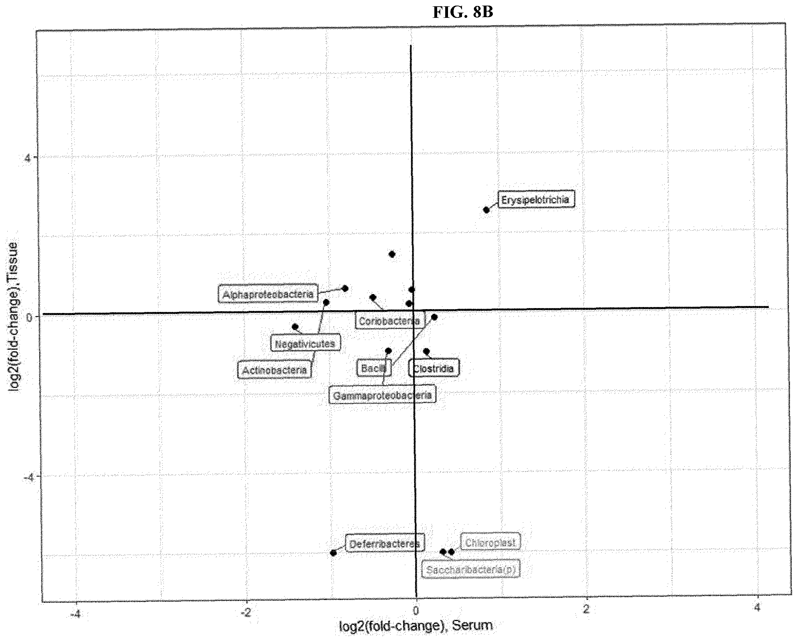

At the class level, *Erysipelotrichia* in a patient was significantly increased in both serum and tissue samples (FIG. 8B).

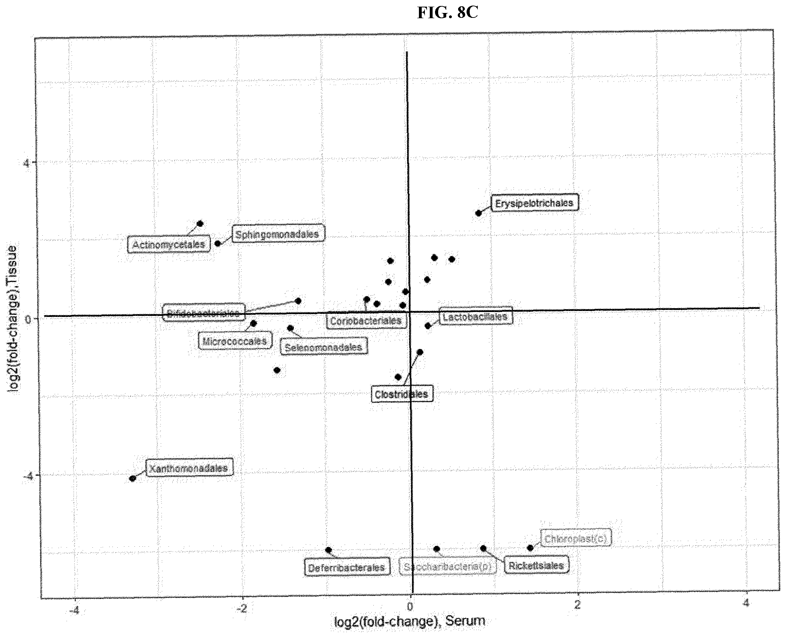

At the order level, *Erysipelotrichiales* was significantly increased in both serum and tissue samples of a patient (FIG. 8C).

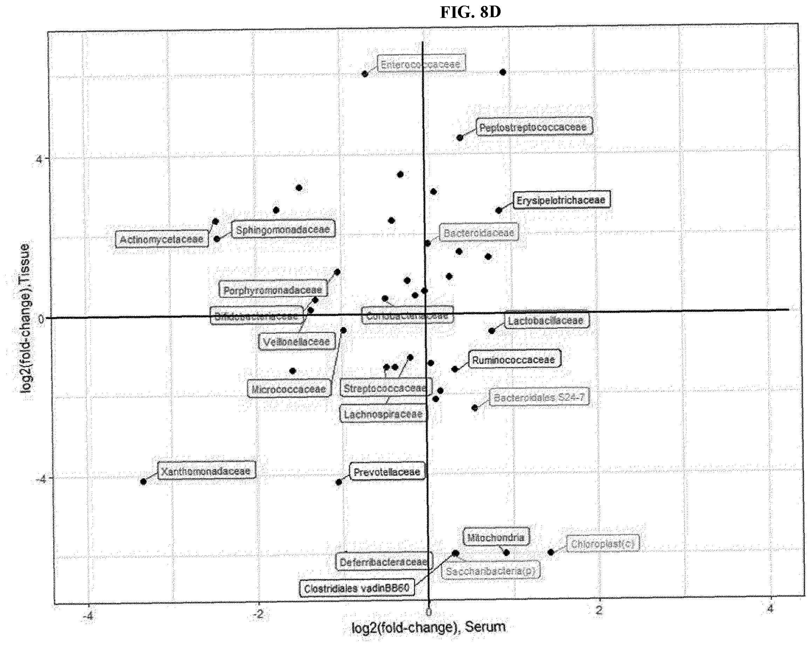

At the family level, in a patient's serum and tissue, *Erysipelotrichaceae* was significantly higher, and *Prevotellaceae* was significantly decreased (FIG. 8D).

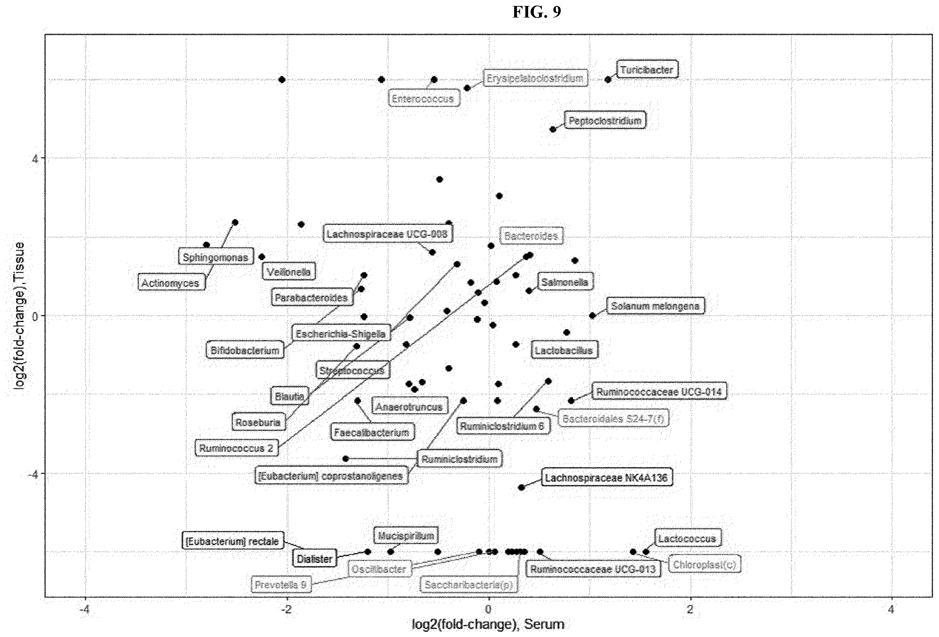

Finally, at the genus level, *[Eubacterium] rectale (E. rectale)* and *Dialister* were significantly decreased in both serum and tissue samples in a patient. In addition, it was confirmed that *Lachnospiraceae* NK4A136 was significantly lower in tissue of a BT patient, but highly increased in serum of the patient compared with the tissue and serum samples of the control (FIG. 9).

### Example 6. Development of BT diagnostic model using blood sample

### Example 6-1. Metagenomic analysis of DNA extracted from blood

To isolate vesicles from blood and extract DNA, first, blood was added to a 10 mL tube and subjected to centrifugation (3,500 x g, 10 min, 4 °C) to precipitate suspended matter, thereby collecting a suspension, followed by transferring the suspension to a new 10 mL tube. Bacteria and impurities were removed from the recovered supernatant using a 0.22-µm filter, transferred to centrifugal filters (50 kD) and centrifuged at 1500 x g and 4 °C for 15 minutes, and then substances smaller than 50 kD were discarded and concentrated up to 10 mL. Again, bacteria and impurities were removed using a 0.22-µm filter, the resulting supernatant was removed through ultracentrifugation using a Type 90ti rotor at 150,000 x g and 4 °C for 3 hours, and the lumpy pellet was suspended with PBS, thereby obtaining vesicles.

100 µl of the bacteria and bacteria-derived vesicles isolated from the stool according to the above-described method was boiled at 100 °C to allow the internal DNA to come out of the lipid and then cooled on ice for 5 mins. Next, the resulting vesicles were centrifuged at 10,000 x g and 4 °C for 30 minutes to remove the remaining suspension, only the supernatant was collected, and then the amount of DNA extracted was quantified using a NanoDrop sprectrophotometer. In addition, to verify whether bacteria-derived DNA was present in the extracted DNA, PCR was performed using 16s rDNA primers shown in Table 1 below, and it was confirmed bacteria-derived DNA was present in the extracted DNA. The results were output as standard flowgram format (SFF) files, and the SFF files were converted into sequence files (.fasta) and nucleotide quality score files using GS FLX software (v2.9), and then credit rating for reads was identified, and portions with a window (20 bps) average base call accuracy of less than 99% (Phred score <20) were removed. After removing the low-quality portions, only reads having a length of 300 bps or more were used (Sickle version 1.33), and, for operational taxonomy unit (OTU) analysis, clustering was performed using UCLUST and USEARCH according to sequence similarity. In particular, clustering was performed based on sequence similarity values of 94% for genus, 90% for family, 85% for order, 80% for class, and 75% for phylum, and phylum, class, order, family, and genus levels of each OTU were classified, and bacteria with a sequence similarity of 97% or more were analyzed (QIIME) using 16S DNA sequence databases (108,453 sequences) of BLASTN and GreenGenes.

### Example 6-2. Brain tumor diagnostic model based on metagenomic analysis of vesicles derived from bacteria isolated from blood

Vesicles were isolated from blood of 170 brain tumor patients and 200 normal individuals, the two groups matched in age and gender, and then metagenomic sequencing was performed thereon using the method of Example 6-1. For diagnostic model development, first, the p value between two groups in the t-test was 0.05 or less, strains with a group average of 0.1% or more were selected, and then AUC, sensitivity, and specificity, which are diagnostic performance indices, were obtained by a logistic regression analysis method.

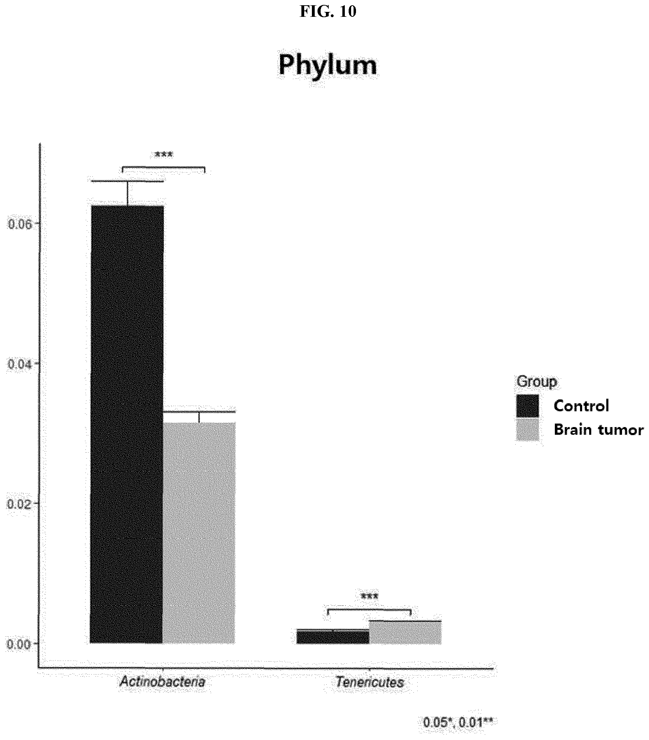

As a result of analyzing vesicles derived from bacteria in blood at a phylum level, a diagnostic model developed using bacteria of the phylum *Actinobacteria,* and the phylum *Tenericutes* as a biomarker exhibited significant diagnostic performance for brain tumor (see Table 3 and FIG. 10).

**[Table 3]**

| | Control | | Brain tumor | | t-test | | | | | |
|---|---|---|---|---|---|---|---|---|---|---|
| Taxon | Mean | SD | Mean | SD | p-value | Rati o | AUC | Accurac y | sensitivity | specifici ty |
| p_Actinoba cteria | 0.0625 | 0.0492 | 0.0316 | 0.0210 | 0.0000 | 0.51 | 0.75 | 0.70 | 0.70 | 0.71 |
| p_Tenericut es | 0.0018 | 0.0039 | 0.0033 | 0.0026 | 0.0000 | 1.86 | 0.72 | 0.64 | 0.85 | 0.46 |

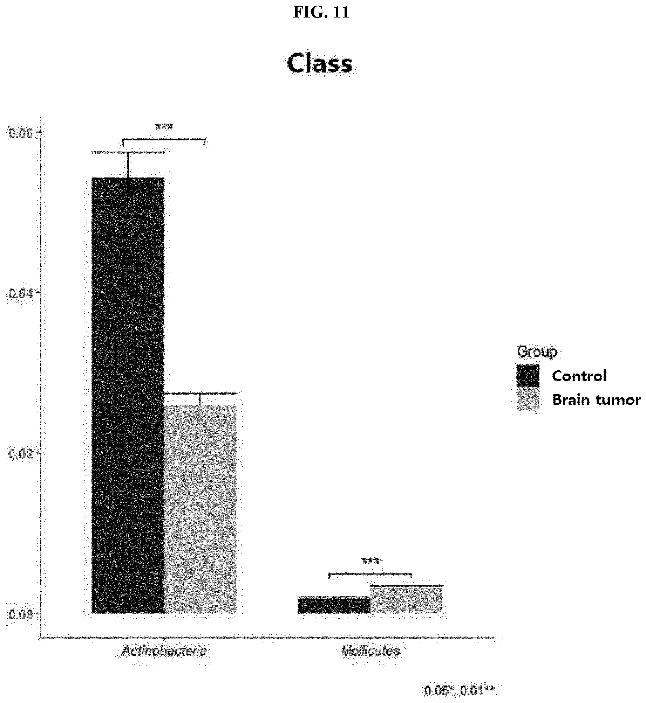

As a result of analyzing vesicles derived from bacteria in blood at a class level, a diagnostic model developed using bacteria of the class *Actinobacteria,* and the class *Mollicutes* as a biomarker exhibited significant diagnostic performance for brain tumor (see Table 4 and FIG. 11).

**[Table 4]**

| | Control | | Brain tumor | | t-test | | | | | |
|---|---|---|---|---|---|---|---|---|---|---|
| Taxon | Mean | SD | Mean | SD | p-value | Rati o | AUC | Accurac y | sensitivity | specifici ty |
| c_Actinoba cteria | 0.0542 | 0.0474 | 0.0258 | 0.0204 | 0.0000 | 0.48 | 0.73 | 0.70 | 0.67 | 0.73 |
| c_Mollicute s | 0.0018 | 0.0039 | 0.0033 | 0.0026 | 0.0000 | 1.86 | 0.72 | 0.64 | 0.85 | 0.46 |

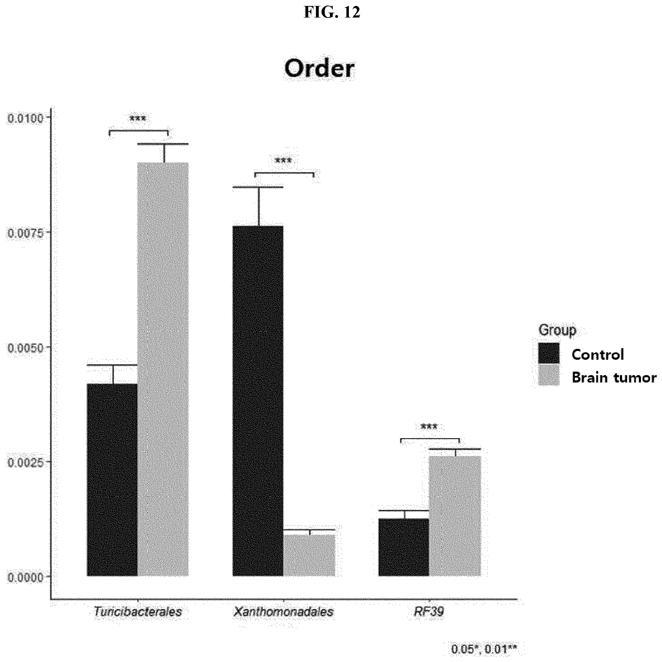

As a result of analyzing vesicles derived from bacteria in blood at an order level, a diagnostic model developed using bacteria of the order *Turicibacterales,* the order *Xanthomonadales,* and the order RF39 as a biomarker exhibited significant diagnostic performance for brain tumor (see Table 5 and FIG. 12).

**[Table 5]**

| | Control | | Brain tumor | | t-test | | | | | |
|---|---|---|---|---|---|---|---|---|---|---|
| Taxon | Mean | SD | Mean | SD | p-value | Rati o | AUC | Accurac y | sensitivity | specifici ty |
| o_Turicibac terales | 0.0042 | 0.0060 | 0.0090 | 0.0055 | 0.0000 | 2.15 | 0.76 | 0.59 | 0.76 | 0.46 |
| o_Xanthom onadales | 0.0076 | 0.0123 | 0.0009 | 0.0017 | 0.0000 | 0.12 | 0.73 | 0.74 | 0.64 | 0.83 |
| o_RF39 | 0.0013 | 0.0027 | 0.0026 | 0.0022 | 0.0000 | 2.07 | 0.70 | 0.59 | 0.85 | 0.39 |

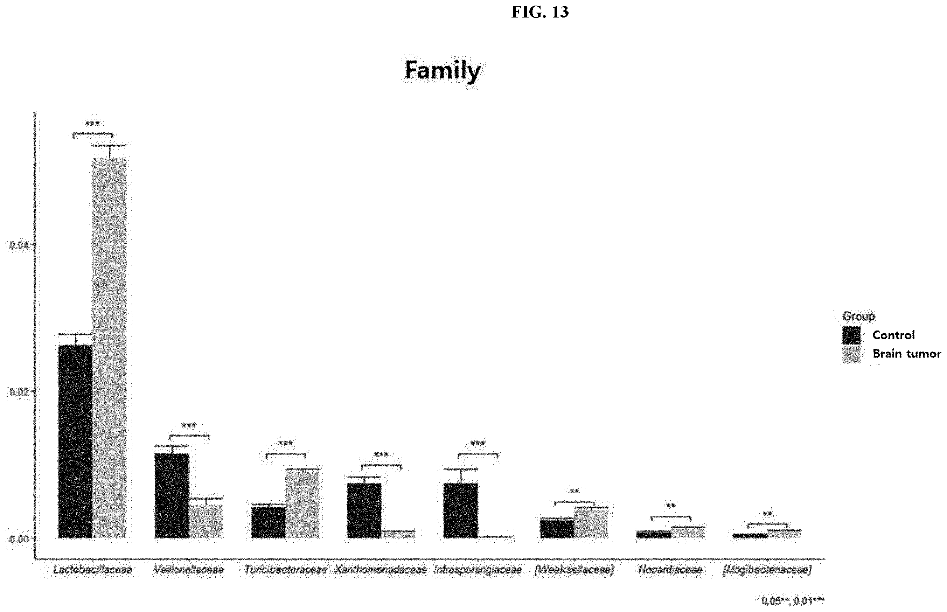

As a result of analyzing vesicles derived from bacteria in blood at a family level, a diagnostic model developed using bacteria of the family *Intrasporangiaceae,* the family *Lactobacillaceae,* the family *Mogibacteriaceae,* the family *Turicibacteraceae,* the family *Nocardiaceae,* the family *Xanthomonadaceae,* the family *Weeksellaceae,* and the family *Veillonellaceae* as a biomarker exhibited significant diagnostic performance for brain tumor (see Table 6 and FIG. 13).

**[Table 6]**

| | Control | | Brain tumor | | t-test | | | | | |
|---|---|---|---|---|---|---|---|---|---|---|
| Taxon | Mean | SD | Mean | SD | p-value | Rati o | AUC | Accurac y | sensitivity | specifici ty |
| f Intrasporangi aceae | 0.0075 | 0.0278 | 0.0002 | 0.0006 | 0.0003 | 0.03 | 0.77 | 0.74 | 0.48 | 0.95 |
| f Lactobacillac eae | 0.0263 | 0.0217 | 0.0516 | 0.0228 | 0.0000 | 1.97 | 0.76 | 0.62 | 0.73 | 0.54 |
| f_[Mogibacteri aceae] | 0.0005 | 0.0017 | 0.0010 | 0.0021 | 0.0157 | 1.96 | 0.76 | 0.51 | 0.94 | 0.17 |
| f Turicibactera ceae | 0.0042 | 0.0060 | 0.0090 | 0.0055 | 0.0000 | 2.15 | 0.76 | 0.59 | 0.76 | 0.46 |
| f_Nocardiaceae | 0.0008 | 0.0024 | 0.0014 | 0.0023 | 0.0206 | 1.69 | 0.74 | 0.49 | 1.00 | 0.07 |
| f Xanthomona daceae | 0.0075 | 0.0119 | 0.0009 | 0.0017 | 0.0000 | 0.12 | 0.73 | 0.77 | 0.67 | 0.85 |
| f [Weeksellace ae] | 0.0025 | 0.0047 | 0.0038 | 0.0054 | 0.0137 | 1.53 | 0.71 | 0.50 | 1.00 | 0.10 |
| f Veillonellace ae | 0.0115 | 0.0154 | 0.0045 | 0.0106 | 0.0000 | 0.39 | 0.71 | 0.58 | 0.73 | 0.46 |

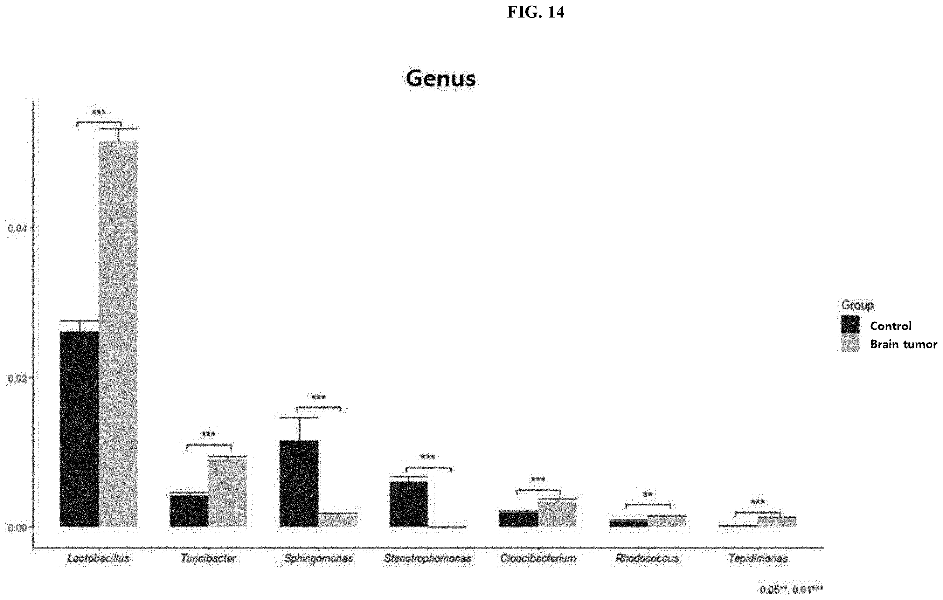

As a result of analyzing vesicles derived from bacteria in blood at a genus level, a diagnostic model developed using bacteria of the genus *Stenotrophomonas,* the genus *Lactobacillus,* the genus *Turicibacter,* the genus *Tepidimonas,* the genus *Sphingomonas,* the genus *Rhodococcus,* and the genus *Cloacibacterium* as a biomarker exhibited significant diagnostic performance for brain tumor (see Table 7 and FIG. 14).

**[Table 7]**

| | Control | | Brain tumor | | t-test | | | | | |
|---|---|---|---|---|---|---|---|---|---|---|
| Taxon | Mean | SD | Mean | SD | p-value | Rati o | AUC | Accurac y | sensitivity | specifici ty |
| g_Stenotroph omonas | 0.0060 | 0.0111 | 0.0001 | 0.0003 | 0.0000 | 0.01 | 0.79 | 0.78 | 0.64 | 0.90 |
| g_Lactobacill | 0.0261 | 0.0216 | 0.0514 | 0.0228 | 0.0000 | 1.97 | 0.76 | 0.62 | 0.73 | 0.54 |
| us | | | | | | | | | | |
| g_Turicibacte r | 0.0042 | 0.0060 | 0.0090 | 0.0055 | 0.0000 | 2.15 | 0.76 | 0.59 | 0.76 | 0.46 |
| g_Tepidimon as | 0.0002 | 0.0009 | 0.0011 | 0.0024 | 0.0000. | 4.79 | 0.76 | 0.49 | 0.91 | 0.15 |
| g_Sphingomo nas | 0.0116 | 0.0440 | 0.0015 | 0.0044 | 0.0016 | 0.13 | 0.75 | 0.65 | 0.55 | 0.73 |
| g_Rhodococc us | 0.0008 | 0.0024 | 0.0014 | 0.0024 | 0.0220 | 1.69 | 0.74 | 0.49 | 1.00 | 0.07 |
| g_Cloacibact erium | 0.0019 | 0.0040 | 0.0034 | 0.0052 | 0.0030 | 1.76 | 0.74 | 0.51 | 0.94 | 0.17 |

Table 8 below simply shows the result of analysis of vesicles derived from bacteria in serum, blood and brain tissue samples.

**[Table 8]**

| **Increase/decrease in BT patients compared to normal control** | | **Increase** | **Decrease** |
|---|---|---|---|
| **Serum** | Phylum | Firmicutes | Actinobacteria, Proteobacteria |
| **Serum** | Class | Clostridia, Bacill, Erysipelotrichia | Gammaproteobacteria, Actinobacteria, Alphaproteobacteria, Negativicutes |
| **Serum** | Order | Clostridiales, Bifidobacteriales, Erysipelotrichales | Lactobacillales, Micrococcales, Sphingomonadales, Selenomonadales |
| **Serum** | Family | Ruminococcaceae, Lactobacillaceae, Peptostreptococcaceae, Erysi pelotrichaceae | Lachnospiraceae, Streptococcaceae, Bifidobacteriaceae, Sphingomonadaceae, Porphyromonadaceae |
| **Serum** | Genus | Ruminococcaceae UCG-014, Lachnospiraceae NK4A136, Ruminococcaceae UCG-013, Lactobacillus, Ruminiclostridium 6, Peptoclostridium | [Eubacterium] coprostanoligenes, Escherichia-Shigella, Blautia, Bifidobacterium, Streptococcus, Sphingomonas |
| **Tissue** | Phylum | Firmicutes, Bacteroidetes, Actinobacteria, Proteobacteria, | Cyanobacteria, Saccharibacteria |
| **Tissue** | Class | Erysipelotrichia | Clostridia, Saccharibacteria (p), Chloroplast |
| **Tissue** | Order | Erysipelotrichales | Clostridiales, Chloroplast (c), Saccharibacteria (p) |
| **Tissue** | Family | Bacteroidaceae Erysi pelotrichaceae | Ruminococcaceae, Bacteroidales S24-7 group, Chloroplast (c), Prevotellaceae, Saccharibacteria (p) |
| **Tissue** | Genus | Bacteroides, Erysi pelatoclostridium | Bacteroidales S24-7 group, Chloroplast (c), Lachnospiraceae NK4A136 group, Prevotella 9, Candidatus Saccharimonas |
| **Blood** | Phylum | Tenericutes | Actinobacteria |
| **Blood** | Class | Mollicutes | Actinobacteria |
| **Blood** | Order | Turicibacterales | Xanthomonadales |
| **Blood** | Family | Lactobacillaceae, Mogibacteriaceae, Turicibacteraceae, Nocardiaceae, Weeksellaceae, | *Intrasporangiaceae,* Xanthomonadaceae, *Veillonellaceae* |
| **Blood** | Genus | Lactobacillus, Turicibacter, Tepidimonas, Rhodococcus, Cloacibacterium | Stenotrophomonas, Sphingomonas |

The above description of the present invention is provided only for illustrative purposes, and it will be understood by one of ordinary skill in the art to which the present invention pertains that the invention may be embodied in various modified forms without departing from the spirit or essential characteristics thereof. Thus, the embodiments described herein should be considered in an illustrative sense only and not for the purpose of limitation.

### [Industrial Applicability]

By diagnosing the risk of the onset of a brain tumor early through the metagenomic analysis of extracellular vesicles derived from bacteria using a human-derived sample according to the present invention, it is possible to diagnose and predict a risk group for brain tumors early, and also to delay or prevent the onset of the disease through appropriate management. In addition, there is industrial applicability in that, even after the onset of a brain tumor, early diagnosis may be performed to reduce the incidence of the brain tumor, a therapeutic effect may not be only increased, but also the disease may be improved and recurrence thereof may be prevented by avoiding exposure to causative factors through metagenomic analysis in patients diagnosed with a brain tumor.

## Claims

1. A method of providing information for diagnosing brain tumor, the method comprising the following processes:
(a) extracting DNAs from extracellular vesicles isolated from a normal individual and a subject sample;
(b) performing polymerase chain reaction (PCR) on the extracted DNA using a pair of primers comprising SEQ ID NO: 1 and SEQ ID NO: 2; and
(c) comparing the increase or decrease in content of extracellular vesicles derived from bacteria of the subject-derived sample with that of the normal individual-derived sample through sequencing of the PCR product.

2. The method of claim 1, wherein the normal individual and subject samples are blood or tissue, and
wherein process (c) comprises comparing an increase or decrease in content of extracellular vesicles derived from one or more bacteria selected from the group consisting of the phylum *Actinobacteria,* the phylum *Proteobacteria,* the phylum *Firmicutes,* the phylum *Bacteroidetes,* the phylum *Cyanobacteria,* the phylum *Saccharibacteria,* and the phylum *Tenericutes,*
extracellular vesicles derived from one or more bacteria selected from the group consisting of the class *Clostridia,* the class *Bacilli,* the class *Erysipelotrichiam,* the class *Gammaproteobacteria,* the class *Alphaproteobacteria,* the class *Negativicutes,* the class *Saccharibacteria (p),* the class *Chloroplast,* the class *Actinobacteria* and the class *Mollicutes,*
extracellular vesicles derived from one or more bacteria selected from the group consisting of the order *Clostridiales,* the order *Bifidobacteriales,* the order *Erysipelotrichales,* the order *Lactobacillales,* the order *Micrococcales,* the order *Sphingomonadales,* the order *Selenomonadales,* the order *Chloroplast (c),* the order *Saccharibacteria (p),* the order *Turicibacterales* and the order *Xanthomonadales,*
extracellular vesicles derived from one or more bacteria selected from the group consisting of the family *Ruminococcaceae,* the family *Peptostreptococcaceae,* the family *Erysipelotrichaceae,* the family *Lachnospiraceae,* the family *Streptococcaceae,* the family *Bifidobacteriaceae,* the family *Sphingomonadaceae,* the family *Porphyromonadaceae,* the family *Bacteroidaceae,* the family *Bacteroidales* S24-7, the family *Chloroplast (c),* the family *Prevotellaceae,* the family *Saccharibacteria (p),* the family *Intrasporangiaceae,* the family *Lactobacillaceae,* the family *Mogibacteriaceae,* the family *Turicibacteraceae,* the family *Nocardiaceae,* the family *Xanthomonadaceae,* the family *Weeksellaceae,* and the family *Veillonellaceae,* or
extracellular vesicles derived from one or more bacteria selected from the group consisting of the genus *Ruminococcaceae* UCG-014, the genus *Lachnospiraceae* NK4A136, the genus *Ruminococcaceae* UCG-013, the genus *Ruminiclostridium* 6, the genus *Peptoclostridium,* the genus *[Eubacterium] coprostanoligenes,* the genus *Escherichia-Shigella,* the genus *Blautia,* the genus *Bifidobacterium,* the genus *Streptococcus,* the genus *Bacteroides,* the genus *Erysipelatoclostridium,* the genus *Bacteroidales* S24-7, the genus *Chloroplast (c),* the genus *Prevotella* 9, the genus *Candidatus Saccharimonas,* the genus *Stenotrophomonas,* the genus *Lactobacillus,* the genus *Turicibacter,* the genus *Tepidimonas,* the genus *Sphingomonas,* the genus *Rhodococcus,* and the genus *Cloacibacterium.*

3. The method of claim 2, wherein the normal individual and subject samples are blood, and
wherein, in process (c), in comparison with the normal individual-derived sample, an increase in the content of the following is diagnosed as brain tumor:
extracellular vesicles derived from bacteria of the phylum *Tenericutes;*
extracellular vesicles derived from bacteria of the class *Mollicutes,*
extracellular vesicles derived from bacteria of the order *Turicibacterales,*
extracellular vesicles derived from one or more bacteria selected from the group consisting of the family *Lactobacillaceae,* the family *Mogibacteriaceae,* the family *Turicibacteraceae,* the family *Nocardiaceae,* and the family *Weeksellaceae,* or
extracellular vesicles derived from one or more bacteria selected from the group consisting of the genus *Lactobacillus,* the genus *Turicibacter,* the genus *Tepidimonas,* the genus *Rhodococcus,* and the genus *Cloacibacterium.*

4. The method of claim 2, wherein the normal individual and subject samples are blood, and
wherein, in process (c), in comparison with the normal individual-derived sample, a decrease in the content of the following is diagnosed as brain tumor:
extracellular vesicles derived from bacteria of the phylum *Actinobacteria,*
extracellular vesicles derived from bacteria of the class *Actinobacteria,*
extracellular vesicles derived from bacteria of the order *Xanthomonadales,*
extracellular vesicles derived from one or more bacteria selected from the group consisting of the family *Intrasporangiaceae,* the family *Xanthomonadaceae,* and the family *Veillonellaceae,* or
extracellular vesicles derived from one or more bacteria selected from the group consisting of the genus *Stenotrophomonas* and the genus *Sphingomonas.*

5. The method of claim 2, wherein the blood is whole blood, serum, plasma, or blood mononuclear cells.

6. The method of claim 5, wherein the normal individual and subject samples are serum, and
wherein, in process (c), in comparison with the normal individual-derived sample, an increase in the content of the following is diagnosed as brain tumor:
extracellular vesicles derived from bacteria of the phylum *Firmicutes,*
extracellular vesicles derived from one or more bacteria selected from the group consisting of the class *Clostridia,* the class *Bacilli,* and the class *Erysipelotrichiam,*
extracellular vesicles derived from one or more bacteria selected from the group consisting of the order *Clostridiales,* the order *Bifidobacteriales,* and the order *Erysipelotrichales,*
extracellular vesicles derived from one or more bacteria selected from the group consisting of the family *Ruminococcaceae,* the family *Lactobacillaceae,* the family *Peptostreptococcaceae,* and the family *Erysipelotrichaceae,* or
extracellular vesicles derived from one or more bacteria selected from the group consisting of the genus *Ruminococcaceae* UCG-014, the genus *Lachnospiraceae* NK4A136, the genus *Ruminococcaceae* UCG-013, the genus *Lactobacillus,* the genus *Ruminiclostridium* 6, and the genus *Peptoclostridium.*

7. The method of claim 5, wherein the normal individual and subject samples are serum, and
wherein, in process (c), in comparison with the normal individual-derived sample, a decrease in the content of the following is diagnosed as brain tumor:
extracellular vesicles derived from one or more bacteria selected from the group consisting of the phylum *Actinobacteria* and the phylum *Proteobacteria,*
extracellular vesicles derived from one or more bacteria selected from the group consisting of the class *Gammaproteobacteria,* the class *Actinobacteria,* the class *Alphaproteobacteria,* and the class *Negativicutes,*
extracellular vesicles derived from one or more bacteria selected from the group consisting of the order *Lactobacillales,* the order *Micrococcales,* the order *Sphingomonadales,* and the order *Selenomonadales,*
extracellular vesicles derived from one or more bacteria selected from the group consisting of the family *Lachnospiraceae,* the family *Streptococcaceae,* the family *Bifidobacteriaceae,* the family *Sphingomonadaceae,* and the family *Porphyromonadaceae,* or
extracellular vesicles derived from one or more bacteria selected from the group consisting of the genus *[Eubacterium] coprostanoligenes,* the genus *Escherichia-Shigella,* the genus *Blautia,* the genus *Bifidobacterium,* the genus *Streptococcus,* and the genus *Sphingomonas.*

8. The method of claim 2, wherein the normal individual and subject samples are tissue, and
wherein, in process (c), in comparison with the normal individual-derived sample, an increase in the content of the following is diagnosed as brain tumor:
extracellular vesicles derived from one or more bacteria selected from the group consisting of the phylum *Actinobacteria,* the phylum *Proteobacteria,* the phylum *Firmicutes,* and the phylum *Bacteroidetes,*
extracellular vesicles derived from bacteria of the class *Erysipelotrichiam,*
extracellular vesicles derived from bacteria of the order *Erysipelotrichales,*
extracellular vesicles derived from one or more bacteria selected from the group consisting of the family *Erysipelotrichaceae* and the family *Bacteroidaceae,* or
extracellular vesicles derived from one or more bacteria selected from the group consisting of the genus *Bacteroides* and the genus *Erysipelatoclostridium.*

9. The method of claim 2, wherein the normal individual and subject samples are tissue, and
wherein, in process (c), in comparison with the normal individual-derived sample, a decrease in the content of the following is diagnosed as brain tumor:
extracellular vesicles derived from one or more bacteria selected from the group consisting of the phylum *Cyanobacteria* and the phylum *Saccharibacteria,*
extracellular vesicles derived from one or more bacteria selected from the group consisting of the class *Clostridia,* the class *Saccharibacteria (p),* and the class *Chloroplast,*
extracellular vesicles derived from one or more bacteria selected from the group consisting of the order *Clostridiales,* the order *Chloroplast (c),* and the order *Saccharibacteria (p),*
extracellular vesicles derived from one or more bacteria selected from the group consisting of the family *Ruminococcaceae,* the family *Bacteroidales* S24-7, the family *Chloroplast (c),* the family *Prevotellaceae,* and the family *Saccharibacteria (p),* or
extracellular vesicles derived from one or more bacteria selected from the group consisting of the genus *Bacteroidales* S24-7, the genus *Chloroplast (c),* the genus *Lachnospiraceae* NK4A136, the genus *Prevotella* 9, and the genus *Candidatus Saccharimonas.*

10. The method of claim 2, wherein the tissue is brain tissue.

11. A method of diagnosing brain tumor, the method comprising the following processes:
(a) extracting DNAs from extracellular vesicles isolated from a normal individual and a subject sample;
(b) performing polymerase chain reaction (PCR) on the extracted DNA using a pair of primers comprising SEQ ID NO: 1 and SEQ ID NO: 2; and
(c) comparing the increase or decrease in content of extracellular vesicles derived from bacteria of the subject-derived sample with that of the normal individual-derived sample through sequencing of the PCR product.

12. A method of providing information for predicting the onset of brain tumor, the method comprising the following processes:
(a) extracting DNAs from extracellular vesicles isolated from a normal individual and a subject sample;
(b) performing polymerase chain reaction (PCR) on the extracted DNA using a pair of primers comprising SEQ ID NO: 1 and SEQ ID NO: 2; and
(c) comparing the increase or decrease in content of extracellular vesicles derived from bacteria of the subject-derived sample with that of the normal individual-derived sample through sequencing of the PCR product.


<110>    MD Healthcare Inc.

<120>    Method for diagnosis of brain tumor using analysis of bacteria
         metagenome

<130>    MPCT20-049

<150>    KR 10-2019-0082317
<151>    2019-07-08

<150>    KR 10-2020-0057634
<151>    2020-05-14

<160>    2

<170>    KoPatentIn 3.0

<210>    1
<211>    50
<212>    DNA
<213>    Artificial Sequence

<220>
<223>    16S_V3_F


<400>    1
tcgtcggcag cgtcagatgt gtataagaga cagcctacgg gnggcwgcag                    50


<210>    2
<211>    55
<212>    DNA
<213>    Artificial Sequence

<220>
<223>    16S_V4_R


<400>    2
gtctcgtggg ctcggagatg tgtataagag acaggactac hvgggtatct aatcc              55